# EUROPEAN PATENT SPECIFICATION

(11) **EP 3 601 596 B1**
(45) Date of publication and mention of the grant of the patent: **03.12.2025**
(21) Application number: 18771954.7
(22) Date of filing: 15.03.2018
(51) Int. Cl.: C12Q 1/6827, C12Q 1/6886

(54) **STRAND-SPECIFIC DETECTION OF BISULFITE-CONVERTED DUPLEXES**
STRANGSPEZIFISCHER NACHWEIS VON BISULFITUMGESETZTEN DUPLEXEN
DÉTECTION SPÉCIFIQUE DES BRINS DES DUPLEX CONVERTIS AU BISULFITE

(30) Priority: 24.03.2017 US 201762476234 P
(43) Date of publication of application: 05.02.2020
(73) Proprietor: The Johns Hopkins University, Baltimore, MD 21218 (US)
(72) Inventor: VOGELSTEIN, Bert, Baltimore, Maryland 21208 (US); KINZLER, Kenneth W., Baltimore, Maryland 21230 (US); PAPADOPOULOS, Nickolas, Towson, Maryland 21204 (US); MATTOX, Austin, Baltimore, Maryland 21218 (US); YEGNASUBRAMANIAN, Srinivasan, Baltimore, Maryland 21218 (US); NELSON, William G., Baltimore, Maryland 21205 (US)
(74) Representative: D Young & Co LLP
(86) International application number: PCT/US2018/022664
(87) International publication number: WO 2018/175202

(56) References cited:
- WO-A1-2017/027653
- WO-A2-2012/142213
- US-A1- 2014 287 404
- US-A1- 2016 194 691
- IVANA BUBANCOVA ET AL: "Next-Generation Sequencing Approach in Methylation Analysis of HNF1B and GATA4 Genes: Searching for Biomarkers in Ovarian Cancer", INTERNATIONAL JOURNAL OF MOLECULAR SCIENCES, vol. 18, no. 2, 22 February 2017 (2017-02-22), pages 474, XP055743245, DOI: 10.3390/ijms18020474
- GARY G CHEN ET AL: "BisQC: an operational pipeline for multiplexed bisulfite sequencing", BMC GENOMICS, BIOMED CENTRAL, vol. 15, no. 1, 16 April 2014 (2014-04-16), pages 290, XP021184280, ISSN: 1471-2164, DOI: 10.1186/1471-2164-15-290
- DIANA L BERNSTEIN ET AL: "The BisPCR2 method for targeted bisulfite sequencing", EPIGENETICS & CHROMATIN, vol. 8, no. 1, 1 August 2015 (2015-08-01), XP055634206, DOI: 10.1186/s13072-015-0020-x
- AUSTIN K. MATTOX ET AL: "Bisulfite-converted duplexes for the strand-specific detection and quantification of rare mutations", PROCEEDINGS OF THE NATIONAL ACADEMY OF SCIENCES, vol. 114, no. 18, 17 April 2017 (2017-04-17), pages 4733 - 4738, XP055742732, ISSN: 0027-8424, DOI: 10.1073/pnas.1701382114
- LEE ET AL.: "NGS-based deep bisulfite sequencing", METHODSX EPUB, vol. 3, 26 November 2015 (2015-11-26), pages 1 - 7, XP055542151
- HOFFMANN ET AL.: "DNA bar coding and pyrosequencing to identify rare HIV drug resistance mutations", NUCLEIC ACIDS RES, vol. 35, no. 13, 2007, pages 1 - 8, XP055059023
- PRIOR ET AL.: "A comprehensive survey of Ras mutations in cancer", CANCER RES, vol. 72, no. 10, 15 May 2012 (2012-05-15), pages 2457 - 2467, XP055117242
- VIDA ARMALIENE: "Thermo Scientific. Phusion U Hot Start DNA Polymerase", THERMO SCIENTIFIC., 23 September 2016 (2016-09-23), XP055542173, Retrieved from the Internet <URL:https://assets.thermofisher.com/TFS-Assets/LSG/manuals/MAN0012916_PhusionUHotStart_DNAPolymerase_F555L_UG.pdf> [retrieved on 20180607]

## Description

### TECHNICAL FIELD

This description is related to the area of nucleic acid analysis. In particular, it relates to nucleic acid sequence analyses which have increased sensitivity and accuracy.

### BACKGROUND

Extensive knowledge of the genetic alterations that underlie cancer is now available, opening new opportunities for the management of patients (1-3). Some of the most important of these opportunities involve "liquid biopsies," i.e., the evaluation of blood and other bodily fluids for mutant DNA template molecules that are released from tumor cells into such fluids. Although the potential value of liquid biopsies was recognized more than two decades ago (4-6), more recent advances in sequencing technology have made this approach practical. For example, it has recently been shown that liquid biopsies of blood can detect minimal amounts of disease in patients with early stage colorectal cancers, thereby providing evidence that could substantially affect their survival (7). Other studies have shown that circulating tumor DNA (ctDNA) can be detected in the blood of patients with other malignancies, as well as in other bodily fluids such as pancreatic cysts, Pap smears, and saliva (8-16).

The vast majority of current technologies for detecting rare mutations employ digital approaches, where each template molecule is assessed, one by one, to determine whether it is wild type or mutant (17). The digitalization can be performed in wells (17), in tiny droplets formed by emulsification or microfluidics (18, 19), or in clusters (20). The most powerful of these approaches employs massively parallel sequencing to simultaneously analyze the entire sequences of hundreds of millions of individually amplified template molecules (21). However, all the currently available sequencing instruments have relatively high error rates, limiting sensitivity at many nucleotide positions to one mutant among 100 wild type (WT) template molecules, even with DNA templates that are of optimal quality (21). The DNA quality of clinical samples is often far less than optimal, compounding the problem. Sensitivity can be increased by pre-treating the DNA to remove damaged bases prior to sequencing (22, 23) and by bioinformatics and statistical methods to enhance base-calls after sequencing (24, 25). Although useful for a variety of purposes, the sensitivity obtainable with these improvements is generally not sufficiently high for the most challenging applications, such as liquid biopsies, which can require detection of one mutant molecule among thousands of WT molecules (9).

Another important way to improve sensitivity is with the use of "molecular barcodes," in which each template is covalently linked to unique identifying sequences (UIDs). Molecular barcodes were originally used to count individual template molecules (26), but were subsequently incorporated into a powerful approach, termed SafeSeqS, for error reduction (27). After incorporation of the UIDs, subsequent amplification steps produce multiple copies of each UID-linked template. Each of the daughter molecules produced by amplification contains the same UID, forming a UID family. To be considered a bona fide mutation, termed a supermutant, every member of the UID family must have the identical sequence at each queried position (27).

There are two general ways to assign molecular barcodes to template DNA molecules. One is used to PCR-amplify specific loci using a set of locus-specific primers, and the other is used to ligate adapters prior to amplification of the entire genome, creating a library. The PCR method uses primers containing a stretch of random (N) bases to distinguish each individual template molecule (exogenous barcodes) (27, 28). The advantage of this approach is that it is applicable to very small amounts of DNA and virtually the only sequences amplified are the desired ones, reducing the amount of sequencing needed to evaluate a specific mutation. The disadvantage is that errors introduced into one strand during the UID-incorporation cycles will create supermutants. This method will still therefore eliminate errors during sequencing, but not errors made during the initial cycles of PCR. The ligation method either employs random sequences in the adapters used for ligation (27-29) or uses the ends of the randomly sheared template DNA to which the adapters are ligated as "endogenous UIDs" (27, 30). Although errors are still introduced during the PCR steps with the ligation approach, its advantage is that both strands can be identified from the sequencing data (duplex sequencing). The probability that the identical, complementary mutation is introduced into both strands is low (the square of the probability of the mutation appearing in only one strand). The disadvantage of this approach is that it requires library preparation and capture of the sequences to be queried, neither of which are highly efficient. WO2012/142213 discloses methods for analyzing nucleic acid sequences which includes assignment of a unique identifier (UID) to each template molecule, amplification to create UID-families and redundant sequencing of the amplification products.

There is a continuing need in the art to sensitively and specifically assay for sequence variations in an efficient manner.

### SUMMARY OF THE INVENTION

The present invention provides a method for detection of a mutation in a population of DNA molecules, comprising:
**treating** a population of DNA molecules with bisulfite to convert unmethylated Cytosine bases in the DNA molecules to Uracil bases, thereby forming a population of converted DNA molecules;
**attaching** molecular barcodes to both strands of the population of converted DNA molecules using an excess of target-specific amplification primers attached to molecular barcodes, thereby forming a population of amplified, barcoded, converted DNA molecules,
   **wherein** said amplification primers comprise at least four primers, each of which is complementary to one of four ends of said converted DNA molecules;
**amplifying** the amplified, barcoded, converted DNA molecules in an amplification reaction to form families of amplified, barcoded, converted DNA molecules, wherein amplified, barcoded, converted DNA molecules that share the same molecular barcode form a family of DNA molecules;
**subjecting** a plurality of members of the families to sequencing reactions to obtain nucleotide sequences for both strands of said plurality of members of the families; and
**comparing** nucleotide sequences of a plurality of members of a family and identifying families in which the members contain a selected mutation, and comparing nucleotide sequences of two complementary strands of an amplified, barcoded, converted DNA molecule and identifying the selected mutation in two complementary strands.

In addition, the present invention provides a method for detecting methylation at a CpG dinucleotide in plus and minus strands simultaneously, comprising:
**treating** a population of DNA molecules with bisulfite to convert unmethylated Cytosine bases in the DNA molecules to Uracil bases, forming a population of converted DNA molecules;
**attaching** molecular barcodes to both strands of the population of converted DNA molecules using an excess of target-specific amplification primers attached to molecular barcodes, forming a population of amplified, barcoded, converted DNA molecules,
**wherein** said amplification primers comprise at least four primers, each of which is complementary to one of four ends of said converted DNA molecules;
**amplifying** the amplified, barcoded, converted DNA molecules in an amplification reaction to form families of amplified, barcoded, converted DNA molecules, wherein amplified, barcoded, converted DNA molecules that share the same molecular barcode form a family of DNA molecules;_
**subjecting** a plurality of members of the families to sequencing reactions to obtain nucleotide sequences for both strands of said plurality of members of the families; and
**comparing** nucleotide sequences of a plurality of members of a family and identifying families in which the members contain a methylated C at a CpG dinucleotide, and
comparing nucleotide sequences of two complementary strands of an amplified, barcoded, converted DNA molecule and identifying the methylated C opposite nucleotide G of the CpG dinucleotide.

According to one aspect of the description a method is provided for detection of rare mutations in a population of DNA molecules. A population of DNA molecules is treated with bisulfite to convert Cytosine bases in the DNA molecules to Uracil bases, forming a population of converted DNA molecules. Molecular barcodes are attached to both strands of the population of converted DNA molecules using an excess of target-specific amplification primers attached to molecular barcodes, forming a population of amplified, barcoded, converted DNA molecules. The amplified, barcoded, converted DNA molecules are amplified in an amplification reaction to form families of amplified, barcoded, converted DNA molecules, wherein amplified, barcoded, converted DNA molecules that share the same molecular barcode form a family of DNA molecules. A plurality of members of the families are subjected to sequencing reactions to obtain nucleotide sequences of both strands of said plurality of members of the families. Nucleotide sequences of a plurality of members of a family are compared and families in which >90% of the members contain a selected mutation are identified. Nucleotide sequences of two complementary strands of an amplified, barcoded, converted DNA molecule are compared and the selected mutation is identified in two complementary strands.

According to another aspect of the description a method is provided for detecting methylation at a CpG dinucleotide in plus and minus strands simultaneously. A population of DNA molecules is treated with bisulfite to convert Cytosine bases in the DNA molecules to Uracil bases, forming a population of converted DNA molecules. Molecular barcodes are attached to both strands of the population of converted DNA molecules using an excess of target-specific amplification primers attached to molecular barcodes, forming a population of amplified, barcoded, converted DNA molecules. The amplified, barcoded, converted DNA molecules are amplified in an amplification reaction to form families of amplified, barcoded, converted DNA molecules, wherein amplified, barcoded, converted DNA molecules that share the same molecular barcode form a family of DNA molecules. A plurality of members of the families is subjected to sequencing reactions to obtain nucleotide sequences of both strands of said plurality of members of the families. Nucleotide sequences of a plurality of members of a family are compared and families in which >90% of the members contain a selected methylated C at a CpG dinucleotide are identified. Nucleotide sequences of two complementary strands of an amplified, barcoded, converted DNA molecule are compared and a methylated C at the CpG dinucleotide is identified in two complementary strands.

In another aspect of the description an amplification primer is provided that comprises a sequence selected from the group consisting of: SEQ ID NO: 1-32.

An additional aspect of the description provides a kit comprising one or more sets of four amplification primers. Each of the primers in one set is complementary to one of four ends of a duplex fragment of bisulfite-converted DNA.

Another aspect of the description is a method for detection of a polymorphism in a population of DNA molecules. A population of DNA molecules is treated with bisulfite to convert Cytosine bases in the DNA molecules to Uracil bases, forming a population of converted DNA molecules. Molecular barcodes are attached to both strands of the population of converted DNA molecules using an excess of target-specific amplification primers attached to molecular barcodes, forming a population of amplified, barcoded, converted DNA molecules. The amplified, barcoded, converted DNA molecules are amplified in an amplification reaction to form families of amplified, barcoded, converted DNA molecules, wherein amplified, barcoded, converted DNA molecules that share the same molecular barcode form a family of DNA molecules. A plurality of members of the families are subjected to sequencing reactions to obtain nucleotide sequences of both strands of said plurality of members of the families. Nucleotide sequences of a plurality of members of a family are compared and families in which >90% of the members contain a selected polymorphism are identified. Nucleotide sequences of two complementary strands of an amplified, barcoded, converted DNA molecule are compared and the selected polymorphism is identified in two complementary strands.

These and other aspects of the description, which will be apparent to those of skill in the art upon reading the specification, provide techniques and tools for sensitively and specifically analyzing DNA variations and modifications.

### BRIEF DESCRIPTION OF THE DRAWINGS

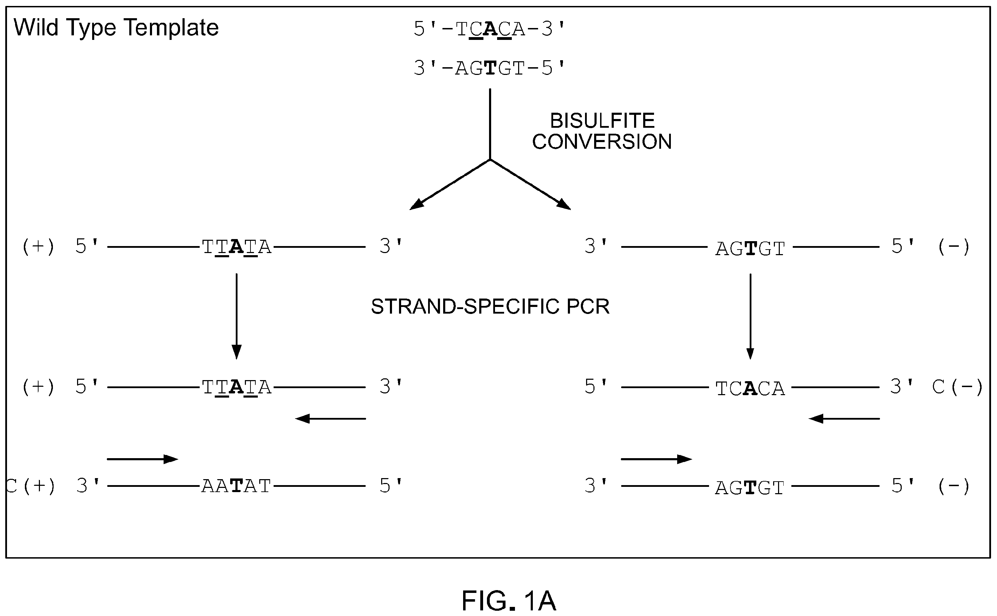

Figs. 1A-1B. Overview of BiSeqS Methodology. Bisulfite conversion creates C > T transitions at unique positions in each strand. Amplification of the (+) and (-) strands with primers that are amplicon and strand-specific allows for targeted amplification and addition of molecular barcodes. Analysis of both strands allows for PCR errors generated in the first PCR cycle to be drastically reduced, as it is highly unlikely a complementary mutation will be generated at the same genomic position on both strands. The conversion and amplification of the Wild Type sequence is presented in panel A, while the conversion and amplification of an A>C transversion is presented in panel B.

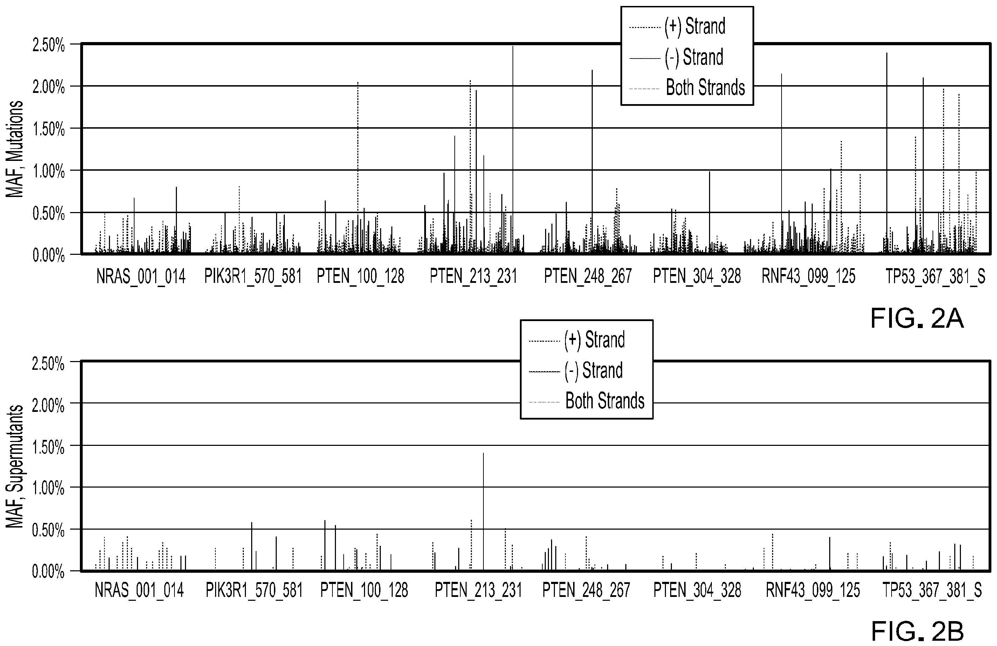

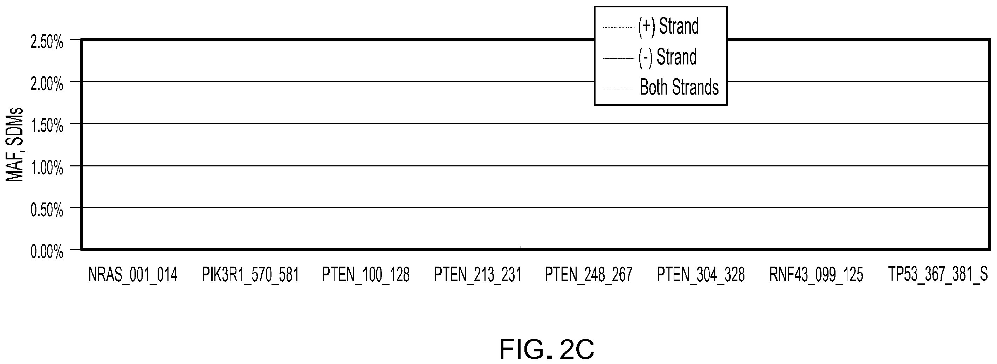

Figs. 2A-2C. BiSeqS drastically reduces the mutant allele frequency (MAF) of single base substitution mutations across amplified loci. MAF of mutations per position across all amplicons (Fig. 2A). MAF of supermutants per position across all amplicons (Fig. 2B). MAF of SDMs per position across all amplicons (Fig. 2C).

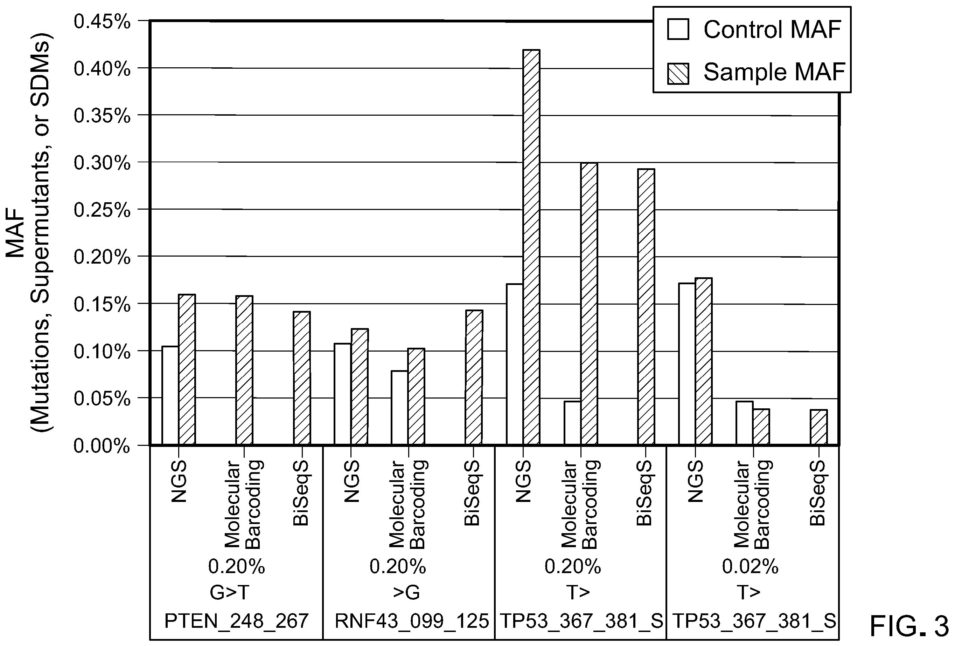

Figure 3. BiSeqS maintains the sensitivity inherent to PCR-based molecular barcoding. Mutant DNA was spiked into normal DNA at a 0.20% or 0.02% target mutant allele frequency and the sequencing data was evaluated by standard NGS, molecular barcoding, and BiSeqS.

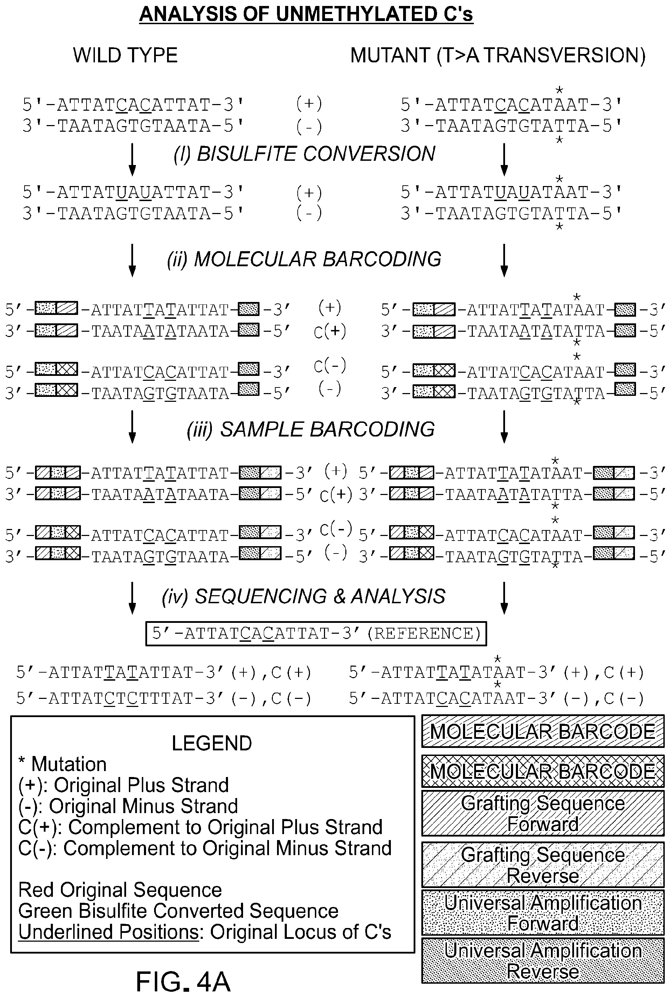

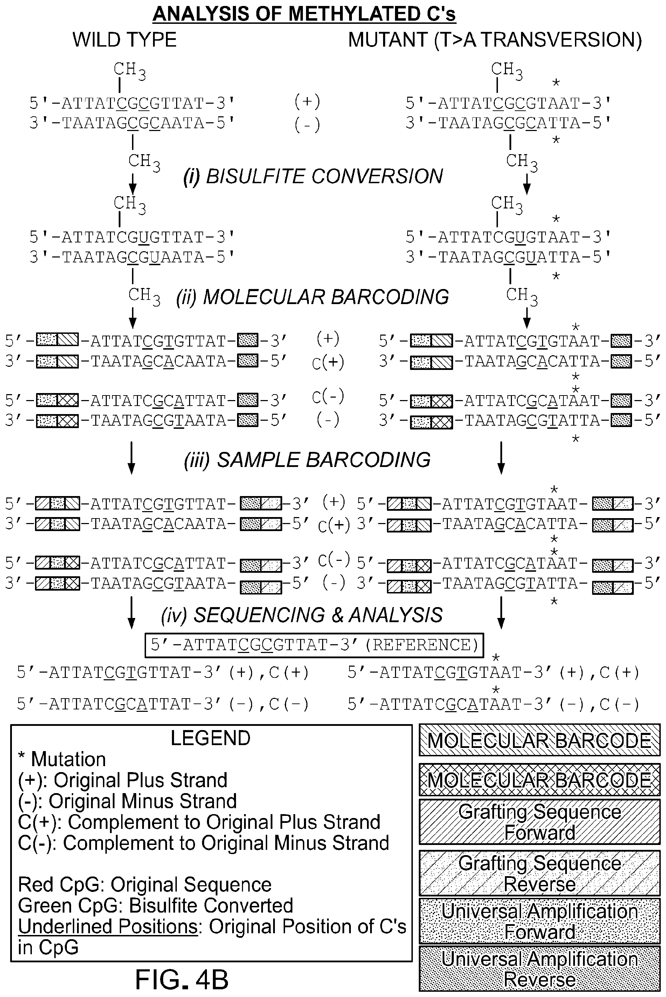

Figs. 4A-4B. (Figure S1.) Detailed schematic of the BiSeqS platform at unmethylated (Fig. 4A) and methylated (Fig. 4B) loci. Unmethylated C are converted to T by bisulfite conversion (Step i), and strand-specific PCR-based molecular barcoding adds unique identifiers to the ends of molecules (Step ii). Sample barcoding (Step iii) amplifies the molecular barcoded DNA, followed by DNA sequencing and analysis (Step iv), which allows for the sequences to be aligned to two reference sequences, one for the (+) strand and one for the (-) strand. Universal amplification primers allow for exponential amplification of all barcoded templates, regardless of the UID sequence. The grafting sequences represent the full-length P5 and P7 sequences required for all paired-end reads on Illumina MiSeq platforms.

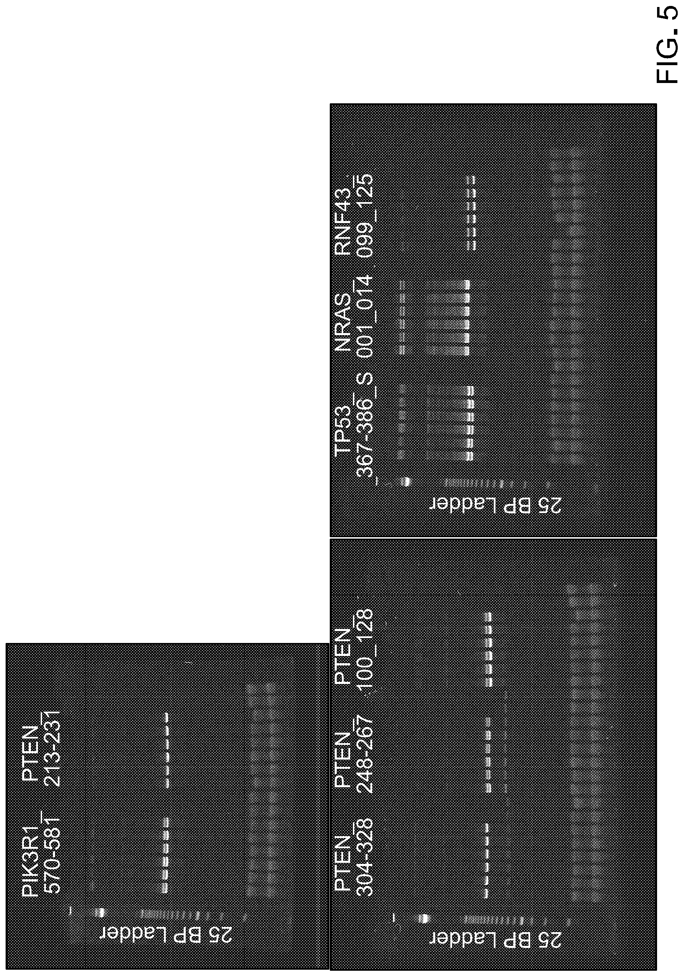

Fig 5. (Figure S2.) Representative examples of BiSeqS amplicons prepared for eight genomic loci. Differences in primer length often create longer products on one strand, allowing for easy discrimination of equimolar amplification of both strands.

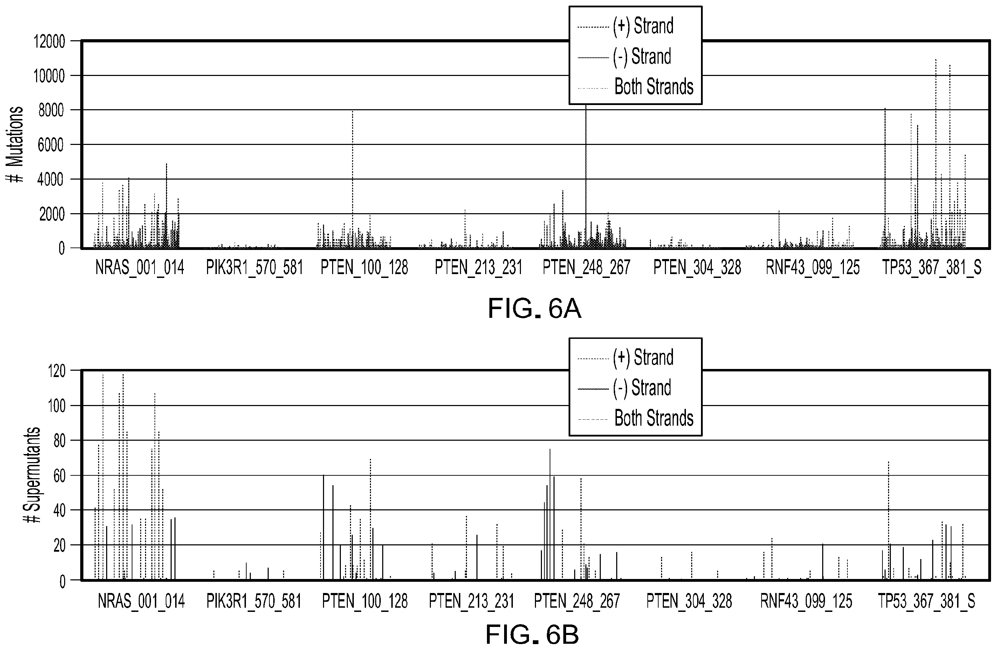

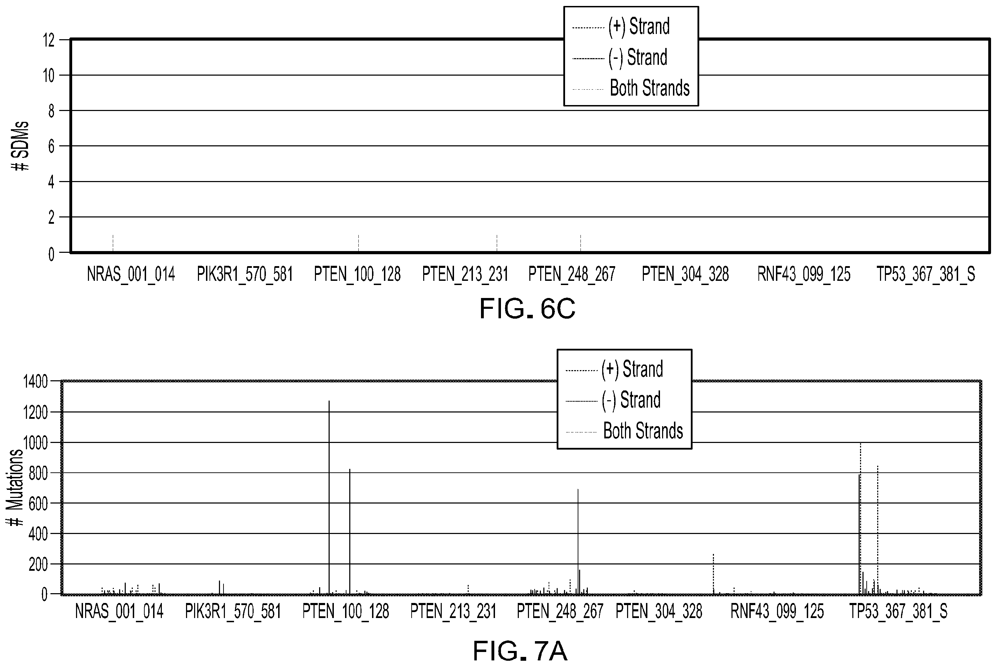

Figs. 6A-6C. (Figure S3.) BiSeqS drastically reduces the number of single base substitution mutations. Number of mutations per position across all amplicons (Fig. 6A). Number of supermutants per position across all amplicons (Fig. 6B). Number of SDMs per position across all amplicons (Fig. 6C). Note that the y-axis scales in panels A & C differ by three orders of magnitude.

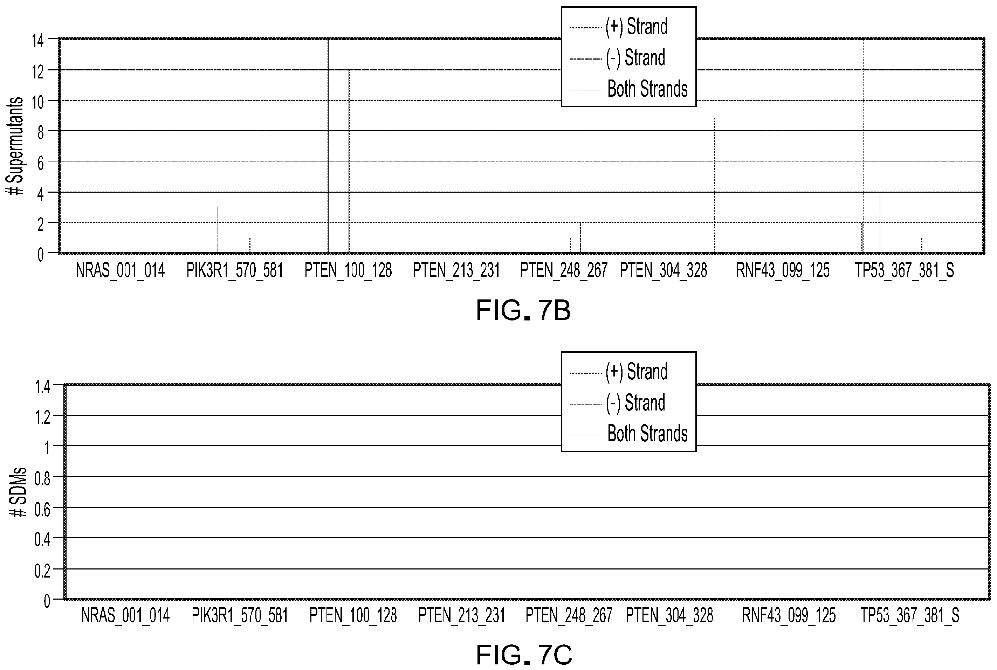

Figs. 7A-7C. (Figure S4.) BiSeqS drastically reduces the number of indel mutations across amplified loci. Number of mutations per position across all amplicons (Fig. 7A). Number of supermutants per position across all amplicons (Fig. 7B). Number of SDMs per position across all amplicons (Fig. 7C).

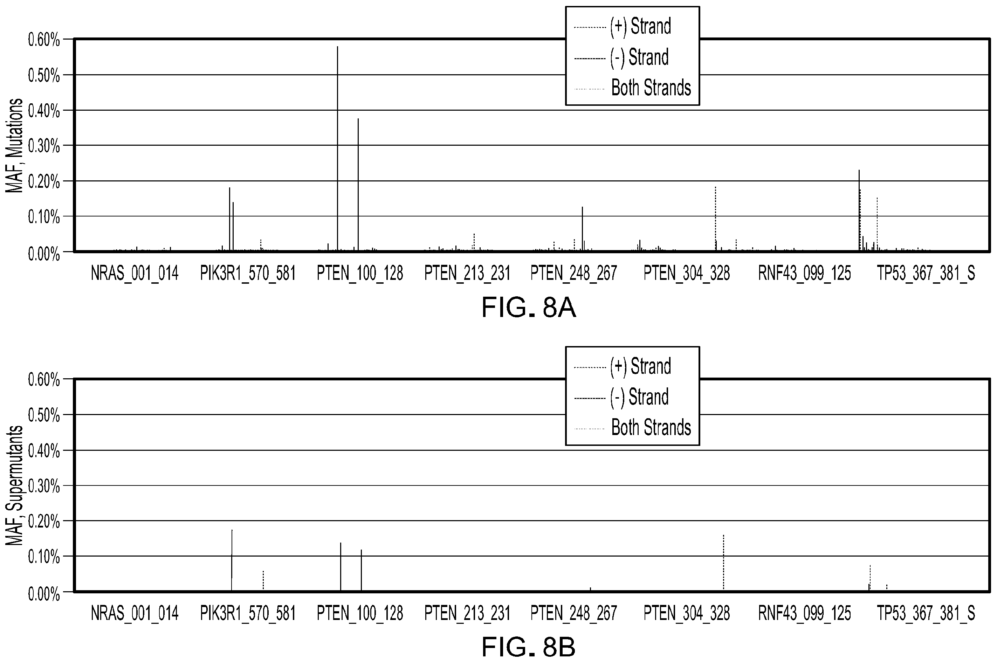

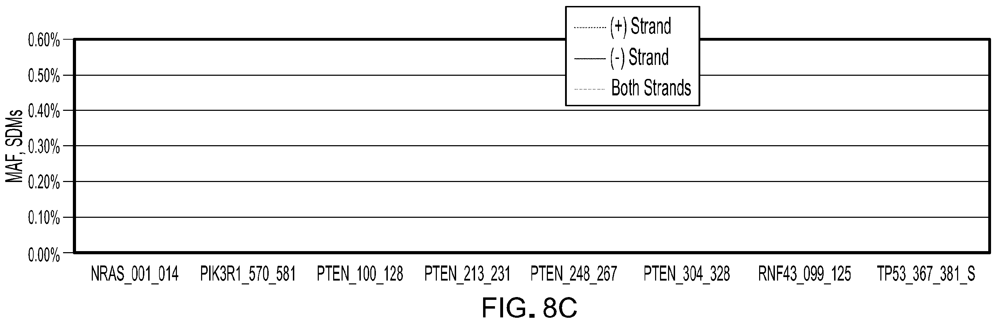

Figs. 8A-8C. (Figure S5.) BiSeqS drastically reduces the mutant allele frequency (MAF) of indel mutations across amplified loci. MAF of mutations per position across all amplicons (Fig. 8A). MAF of supermutants per position across all amplicons (Fig. 8B). MAF of SDMs per position across all amplicons (Fig. 8C).

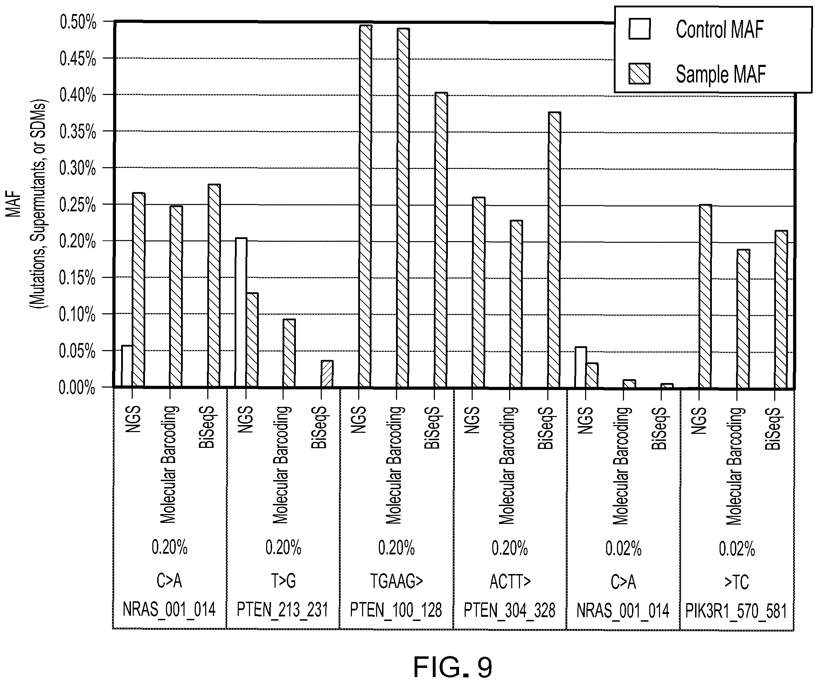

Fig. 9. (Figure S6.) Sensitivity of BiSeqS across all additional amplicons at nominal mutant allele fractions (MAF) of 0.20% and 0.02%. BiSeqS maintains the sensitivity inherent to PCR-based molecular barcoding by detecting mutations at a similar frequency to NGS and molecular-barcode based sequencing.

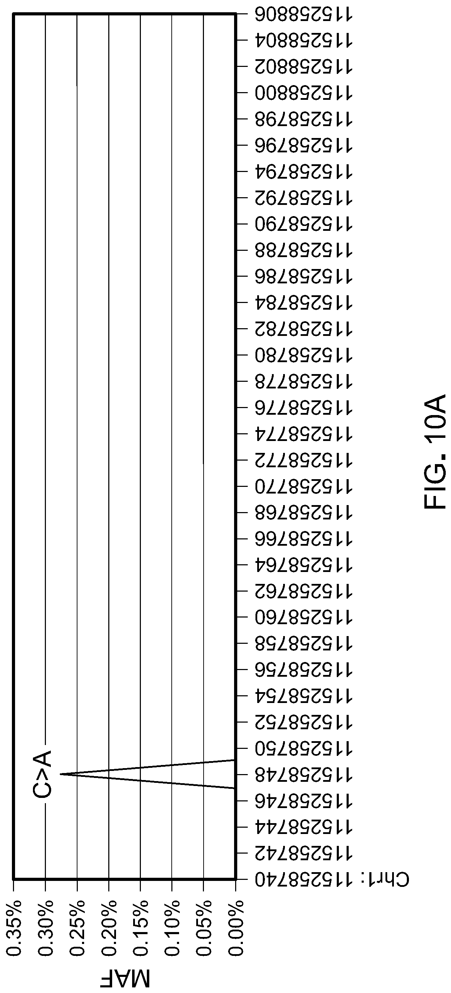

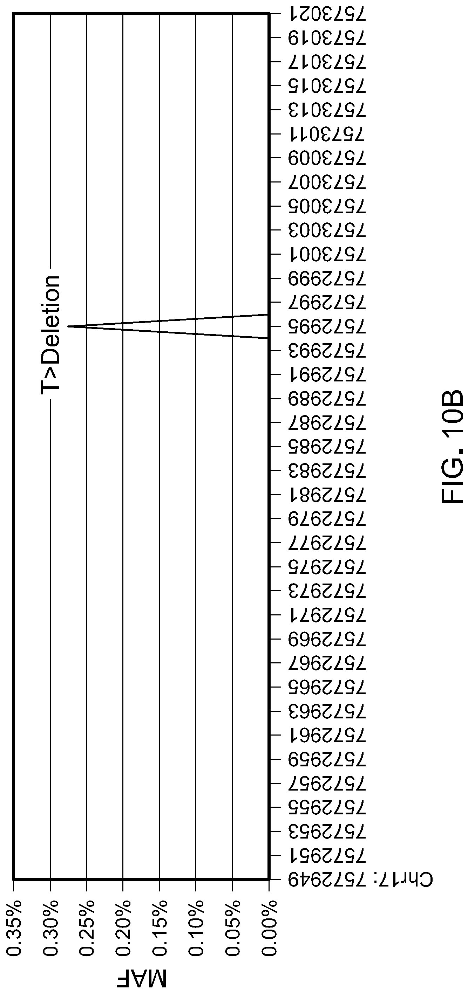

Figs. 10A-10B. (Figure S7.) Signal-to-Noise plots show that BiSeqS allows for the robust detection of double strand mutations. (Fig. 10A) A C>A transversion in *NRAS* at an MAF of 0.20%. (Fig. 10B) A T> deletion in *TP53* at an MAF of 0.20%. The actual mutations at the expected positions are detectable in vast excess over background at the other positions using the BiSeqS method.

### DETAILED DESCRIPTION OF THE INVENTION

The inventors have developed an approach that incorporates advantages of both the PCR- and ligation-based approaches described above. This approach takes advantage of the fact that bisulfite treatment can efficiently convert dC bases in DNA to U bases. This conversion makes the two strands of DNA distinguishable, and was previously used to distinguish RNA transcripts copied from each of the two possible template strands of DNA (31). Bisulfite conversion has also been extensively used to distinguish methylated C-residues, which do not get converted to T bases, from unmethylated C bases, thereby illuminating epigenetic changes (32). It has also been shown that dC bases can be partially converted to T bases so that each individual template DNA molecule can be distinguished from others by its unique pattern of C to T changes, thereby creating an intrinsic barcode similar to what can be achieved with externally added UIDs (33). DNA in which all C bases have been fully converted to T bases can be used as PCR-templates with specially designed primers linked to exogenous barcodes. This allows individual mutations to be assessed on both strands (duplex sequencing) in a reliable manner, without creation of libraries and with a relatively small number of sequencing reads.

The detection of rare mutations in clinical samples in essential to the screening, diagnosis, and treatment of cancer. While next generation sequencing has greatly enhanced the sensitivity of detecting mutations, the relatively high error rate of these platforms limits their overall clinical utility. The elimination of sequencing artifacts could facilitate the detection of early stage cancers and provide improved treatment recommendations tailored to the genetic profile of a tumor. BiSeqS, a bisulfite conversion-based sequencing approach, allows for the strand-specific detection and quantification of rare mutations. BiSeqS eliminates nearly all sequencing artifacts in three common types of mutations and thereby considerably increases the signal-to-noise ratio for diagnostic analyses.

Two types of barcodes are used in BiSeqS. *Molecular* barcodes serve to identify individual template molecules in an original sample prior to barcoding and amplification. Each individual template molecule will have a unique molecular barcode. *Sample* barcodes serve to identify a reaction sample or aliquot of an original sample; all template molecules in the reaction sample or aliquot share a barcode that identifies the reaction sample or aliquot. Barcodes may be, for example, randomly generated nucleotide runs or intentionally chosen nucleotide runs. For attaching molecular barcodes in particular, the number of individual molecular barcodes in a reaction mixture will be in excess of the number of template molecule. In the sequence listing which forms part of this application, barcodes are represented as a string of Ns.

Bisulfite conversion will be close to complete conversion. Thus primer design for amplifying bisulfite converted duplex oligonucleotides utilizes complementarity to the converted sequence. Primers are designed to be used in sets of at least four so that both strands of the original duplex template are amplified, sequenced, and identifiable.

Amplification of barcoded sequences generates families of similarly barcoded templates. Each family shares a molecular barcode, denoting that it derives from a single template molecule. Sequencing of the population of amplified templates, including multiple members of a family, permits comparison of nucleotide sequences of multiple members of a single family and assessment of the fraction of members of a family that contain a particular mutation. A high fraction, such as greater than 50, 60, 70, 80, 90, or 95% of families with a particular mutation suggests that the mutation was present in the original sample, prior to amplification. However, some of the identified mutations may still be ones that have been introduced during processing due to *in vitro* enzymatic errors. Detection of mutations that are due to such errors can be further reduced by comparing sequences obtained from families of two complementary strands. Requiring that a mutation exist on families generated from two strands reduces artifactual apparent mutations significantly.

Fragments of nucleic acids may optionally be obtained using a random fragment forming technique such as mechanical shearing, sonicating, or subjecting nucleic acids to other physical or chemical stresses. Fragments may not be strictly random, as some sites may be more susceptible to stresses than others. Endonucleases that randomly or specifically fragment may also be used to generate fragments. Size of fragments may vary, but desirably will be in ranges between 30 and 5,000 basepairs, between 100 and 2,000 basepairs, between 150 and 1,000 basepairs, or within ranges with different combinations of these endpoints. Nucleic acids may be, for example, RNA or DNA. Modified forms of RNA or DNA may also be used.

Attachment of a molecular barcode to an analyte nucleic acids fragment may be performed by any means known in the art, including enzymatic, chemical, or biologic. One means employs a polymerase chain reaction. Another means employs a ligase enzyme. The enzyme may be mammalian or bacterial, for example. Ends of fragments may be repaired prior to joining using other enzymes such as Klenow Fragment of T4 DNA Polymerase. Other enzymes which may be used for attaching are other polymerase enzymes. A molecular barcode may be added to one or both ends of the fragments, preferably to both ends. A molecular barcode may be contained within a nucleic acid molecule that contains other regions for other intended functionality. For example, a universal priming site may be added to permit later amplification. Another additional site may be a region of complementarity to a particular region or gene in the analyte nucleic acids. A molecular barcode may be from 2 to 4,000, from 100 to 1000, from 4 to 400, bases in length, for example.

Molecular barcodes may be made using random addition of nucleotides to form a short sequence to be used as an identifier. At each position of addition, a selection from one of four deoxyribonucleotides may be used. Alternatively a selection from one of three, two, or one deoxyribonucleotides may be used. Thus the molecular barcodes may be fully random, somewhat random, or non-random in certain positions. Another manner of making molecular barcodes utilizes pre-determined nucleotides assembled on a chip. In this manner of making, complexity is attained in a planned manner.

A cycle of polymerase chain reaction for adding exogenous molecular barcodes refers to the thermal denaturation of a double stranded molecule, the hybridization of a first primer to a resulting single strand, the extension of the primer to form a new second strand hybridized to the original single strand. A second cycle refers to the denaturation of the new second strand from the original single strand, the hybridization of a second primer to the new second strand, and the extension of the second primer to form a new third strand, hybridized to the new second strand. Multiple cycles may be required to increase efficiency, for example, when analyte is dilute or inhibitors are present.

Amplification of fragments containing a molecular barcode can be performed according to known techniques to generate families of fragments. Polymerase chain reaction can be used. Other amplification methods can also be used, as is convenient. Inverse PCR may be used, as can rolling circle amplification. Amplification of fragments typically is done using primers that are complementary to priming sites that are attached to the fragments at the same time as the molecular barcodes. The priming sites are distal to the molecular barcodes, so that amplification includes the molecular barcodes. Amplification forms a family of fragments, each member of the family sharing the same molecular barcode. Because the diversity of molecular barcodes is greatly in excess of the diversity of the fragments, each family should derive from a single fragment molecule in the analyte. Primers used for the amplification may be chemically modified to render them more resistant to exonucleases. One such modification is the use of phosphorothioate linkages between one or more 3' nucleotides. Another employs boranophosphates. Additionally, LNA (locked nucleic acid) bases may be used in the primers; these can increase the Tₘ of an oligonucleotide containing them.

Family members are sequenced and compared to identify any divergences within a family. Sequencing is preferably performed on a massively parallel sequencing platform, many of which are commercially available. If the sequencing platform requires a sequence for "grafting," *i.e.,* attachment to the sequencing device, such a sequence can be added during addition of molecular barcodes or separately. A grafting sequence may be part of a molecular barcoded primer, a universal primer, a gene target-specific primer, the amplification primers used for making a family, a sample barcoded primer, or separate. Redundant sequencing refers to the sequencing of a plurality of members of a single family.

A threshold can be set for identifying a mutation in an analyte. If the "mutation" appears in all members of a family, then it derives from the analyte. If it appears in less than all members, then it may have been introduced during the analysis. Thresholds for calling a mutation may be set, for example, at 1%, 5%, 10%, 20%, 30%, 40%, 50%, 60%, 70%, 80%, 90%, 95%, 97%, 98 %, or 100%. Thresholds will be set based on the number of members of a family that are sequenced and the particular purpose and situation.

Mutations which are detected, monitored, and/or analyzed according to the methods disclosed here may be in cancer driver genes or cancer passenger genes. They may be in other disease-causing or disease-related genes. They may simply be somatic mutations or germline polymorphisms that have no known functional consequence. Examples of driver genes which may be analyzed include *NRAS, PIK3R1, PTEN, RNF43,* and *TP53.* But the methods are in no way limited to these genes. Similarly, the method can be used to detect methylation on both strands of a duplex nucleic acid molecule.

Polymerases which can be used for amplification steps of the method can be any that have properties that are desirable for a particular amplification. We used ThermoFisher Phusion U Hot Start^{™} polymerase in the examples, but we also tested other polymerases and combinations of enzymes. These included Enzo AMPIGENE HS TAQ^{™} Polymerase; BioRad iTAQ Hot Start DNA Polymerase^{™}; ThermoFisher Phusion HotStart II DNA^{™} Polymerase; and Sigma Aldrich FastStart^{™} DNA Polymerase and combinations of these mentioned polymerases.

Amplification primers may be packaged separately or in combinations. They may be in a liquid or dried. The package or kit may optionally contain analytic information on the primers and/or instructions for carrying out methods according to the invention. Kits may optionally contain additional components, such as polymerase enzyme(s), amplification buffer(s), reaction vessels, or other tools to facilitate practice of the methods.

The results described in the examples show that BiSeqS can accurately quantify rare mutations in a highly sensitive and specific manner. We envision that its major use will be in the surveillance of patients with cancer whose primary tumors have been sequenced. It has already been shown that liquid biopsies can be used for this purpose and can accurately identify patients whom are in clinical remission but are destined to recur (7, 11, 44). Many such patients, particularly when their residual burden of disease is small and therefore most likely to be cured by adjuvant therapy (45), have only one or two mutant DNA molecules in 10 ml of plasma. In such situations, a technique like BiSeqS, which can efficiently use all template molecules while maintaining high specificity, could prove particularly useful.

A disadvantage of BiSeqS is that it cannot be applied to most transition mutations because of the ambiguities caused by the bisulfite conversion of C to U, mimicking such transitions. Although one strand is still susceptible to BiSeqS, the power of the technology lies in its ability to detect mutations in both strands, so it poses no advantages over molecular barcoding for such mutations. For example, single base substitutions in *KRAS* codons 12, 13, and 61 are commonly mutated in colon, rectal, and pancreatic adenocarcinomas (46). BiSeqS can be used to quantify *KRAS* mutations in 38.7%, 43.4%, and 47.6% of these cancers, respectively (47). Across all cancers and mutations cataloged in the IARC *TP53* database, approximated 44% of all mutations (i.e. SBS and indels) are amenable to BiSeqS analysis (IARC TP53 Database, R18).

Additionally, bisulfite treatment can result in conversion of methylated C bases to U in rare instances, depending upon the incubation time and reagent concentration (48). The protocol used for BiSeqS employs reduced incubation temperatures that appear to minimize this possibility (48), but sequence heterogeneity at methylated CpG sites may raise background and such sites are not preferred for mutation evaluation.

However, for liquid biopsies in surveillance, limitations inherent to a single gene are not a major issue because several different mutations, including transversions and indels, are generally observed upon genome-wide sequencing of cancers (1-3), and any identified mutation could in principle be applied to this clinical scenario. A recent study of 3,281 cancer samples highlighted that 93% had at least one non-synonymous mutation in at least one driver gene (49). While the average number of point mutations and small indels varied across tumor types, most cancers have at least one driver gene mutation that should be amenable to BiSeqS analysis (49). It is also worth noting that passenger gene mutations that are clonal can also be useful for diagnostic evaluation (50). Because there are at least 10-fold as many passenger mutations as driver gene mutations in nearly all cancers, it is likely that the vast majority of cancers will have several somatic mutations that could be assessed by BiSeqS. For example, in a study of 1157 single base substitutions detected in breast cancer, we calculate that 54.7% of substitutions would be amenable to BiSeqS analysis, in addition to the 7.4% of the tumors that contain insertion or deletion mutations, for a total of 62.1% of tumors (51).

The power of BiSeqS lies in its ability to drastically reduce background errors. Thus, BiSeqS may also complement screening for other genomic alterations, such as structural variants (SV), for rare allele detection and monitoring (52). Structural variants (SVs) provide exquisitely specific markers for cancer that can be used for liquid biopsies (9, 50). Simple polymerase errors do not produce structural variants, providing advantages over single base substitutions as diagnostic targets. On the other hand, there are disadvantages to the use of SVs as diagnostic markers. First, SV detection requires whole genome sequencing of tumors, rather than targeted sequencing of tumors, for their initial detection; the latter is currently much less expensive than the former. Second, and more importantly, structural variants are "private," *i.e.,* generally confined to one or a small number of patients. To be employed as a tumor marker, primers that specifically amplify the translocation junction must be designed and tested on the patient's tumor to ensure that the structural variant is somatic and the amplicon is specific. Although this approach is feasible in a research setting, it is not easily practicable in large scale settings. In contrast, single base substitutions and indels in driver genes are observed in numerous independent tumors, and a small set of "off-the-shelf" primers can be used to assess most patients. For example, we estimate that >98% of patients with colorectal cancer have mutations detectable through amplification with one of 130 pre-designed primer pairs.

In the future, it is possible that chemical treatments of DNA that convert A:T bp (rather than C:G) bp to other bp could substitute for bisulfite when transition mutations must be analyzed. Another avenue for future research is multiplexing, permitting mutations in a variety of amplicons to be assessed simultaneously in screening scenarios. This multiplexing is more difficult than normal because two amplicons must be designed for each region of interest while achieving homogeneous efficiency of every amplicon in all regions of interest.

The above disclosure generally describes the present invention. A more complete understanding can be obtained by reference to the following specific examples.

### EXAMPLE 1

### MATERIALS & METHODS

Briefly, DNA from macro-dissected formalin-fixed paraffin-embedded (FFPE) tumor sections was extracted and bisulfite treated with an EZ DNA Methylation Kit (Zymo Research, Cat. # D5001). Custom primers containing a unique identifier (UID) and amplicon-specific sequence were used to amplify both strands of DNA, and the resulting products were sequenced on an Illumina MiSeq instrument. To characterize the specificity of BiSeqS, DNA isolated from one normal tissue was bisulfite-treated and processed through the BiSeqS pipeline to query for single base substitutions and indels. To characterize the sensitivity of BiSeqS, macro-dissected tumor samples with known MAFs were diluted with the DNA from normal WBCs to obtain final neoplastic cell contents ranging from 0.02% to 0.20%, bisulfite-treated and processed through the BiSeqS pipeline. More details are provided below.

### Human Tissues

Formalin-fixed paraffin-embedded (FFPE) tumor sections were macro-dissected under a dissecting microscope to ensure a neoplastic cellularity of >30%. DNA was purified with a Qiagen FFPE Kit (Qiagen, Cat. # 56494). Tumor samples with known MAFs were diluted with the DNA from normal WBCs to obtain final neoplastic cell contents ranging from 0.02% to 0.20%. To precisely quantify the DNA concentrations of the tumor and normal DNA samples, various mixtures of tumor and normal DNA were amplified with primers that revealed normal single nucleotide polymorphisms within the final amplicons. NGS was then used to quantify the fraction of neoplastic cells within each of the tested mixtures, and the same mixtures were then used as template DNA for BiSeqS, as described below. All tissues were obtained from consented patients at the Johns Hopkins Hospital with the approval of the Johns Hopkins Institutional Review Board.

### Bisulfite Treatment and PCR Amplification of Purified DNA for BiSeqS

After extensive testing of various commercially available bisulfite conversion kits, we chose the EZ DNA Methylation Kit (Zymo Research, Cat. # D5001) to bisulfite treat and desulphonate purified DNA samples following the manufacturer's recommended protocol. DNA was eluted in 10 µL of Elution Buffer and stored at -20°C. Custom HPLC-purified PCR Primers (IDT) were designed for each bisulfite-converted strand of the DNA double helix at the amplified loci (sequence listing). Compared to traditional PCR primers, the custom primers were longer to account for the reduced sequence complexity of bisulfite-converted DNA. Each forward primer contained the sequence necessary for well barcode amplification at the 5' end, followed by a string of 14 random nucleotides that served as the unique identifier (UID), and amplicon-specific primer sequences at the 3' end (Fig. 4A and 4B). Each reverse primer contained the sequence necessary for well barcode amplification at the 5' end, followed by amplicon-specific primer sequences. To anneal to bisulfite-converted DNA, it is important to replace specific nucleotides in the various wild type amplicon-specific primer sequences. T replaced C in the plus strand forward primer, while A replaced G in the plus strand reverse primer. A replaced G in the minus strand forward primer, and T replaced C in the minus strand reverse primer.

The molecular barcoding PCR cycles included 12.5 µL of 2X Phusion U Hot Start PCR Master Mix (ThermoFisher, Cat. # F533S) in a 25 µL reaction, and optimized concentrations of each forward and reverse primer, ranging from 0.125 µM to 4 µM of each forward and each reverse primer for a total of four primers per well. The following cycling conditions were used: one cycle of 95°C for 3 minutes, 20 cycles of 95°C for 10 seconds, 63°C for 2 minutes, and 72°C for 2 minutes.

AMPure XP (Beckman Coulter, Cat. # A63881) was used to remove the primers for UID assignment. 0.025% of the PCR product generated from the UID cycles was used for the well barcoding (WBC) cycles. Primers used for the well barcode step were identical to those described previously and are diagrammed in Fig. 4A and 4B (28). The WBC cycles were performed in 25 µL reactions containing 11.8 µL of water (ThermoFisher UltraPure, Cat. # 10977-023), 5 µL of 5X Phusion HF Buffer (ThermoFisher, Cat. # F518L), 0.5 µL of 10 mM dNTPs (NEB, Cat. # N0447L), and 0.25 µL of Phusion Hot Start II DNA Polymerase (ThermoFisher, Cat. # F549L). The following cycling conditions were used: one cycle of 98°C for 2 minutes, 24 cycles of 98°C for 10 seconds, 65°C for 2 minutes, and 72°C for 2 minutes.

### Sequencing

Sequencing of all the amplicons described above was performed using an Illumina MiSeq instrument. The total length of the reads used for each instrument varied from 79 to 130 bases. Reads passing Illumina CASAVA Chastity filters were used for subsequent analysis.

### BiSeqS Pipeline

High quality reads were processed with the SafeSeqS pipeline (28) to generate aligned data that were then organized into tables for each BiSeqS analysis. Each of the tables contains: (i) strand information, (ii) well barcode and UID sequences, (iii) information listing all differences from the reference amplicon, and (iv) prevalence of each UID family corresponding to a change with respect to all UID families per amplicon. To determine whether a combination of plus and minus strand changes constitute a double strand mutant, the various mutations detected at a specific genomic locus are compared with respect to: (i) sample identity, (ii) chromosome, (iii) genomic position, and (iv) mutation type. Changes were called as true mutations when: (i) the change appeared on both the plus and the minus strands, and (ii) when the MAFs corresponding to the plus and minus strands differed by less than 10-fold.

### Characterization of BiSeqS Specificity

To characterize the specificity of BiSeqS, DNA isolated from one normal tissue was bisulfite-treated and processed through the BiSeqS pipeline to query for single base substitutions and indels. Analysis using NGS across 8 amplicons and 608 bases for indels yielded 907 unique mutations were identified on the plus strand and 958 unique mutations were identified on the minus strand that were ultimately amenable to analysis by BiSeqS. For each strand of each amplicon, we calculated the mutant allele frequency (MAF) by dividing the number of reads or the number of UIDs containing > 2 mutant reads per UID (UID Family Count > 2) by the number of total reads or the number of total UIDs, respectively. Using molecular barcodes to group reads into families decreased the number of unique mutations to 92 on the plus strand and 71 on the minus strand (data not shown). After matching the plus and minus strand amplicons and imposing a filter of less than 10 for the ratio of mutations observed on the plus strand to the ratio of mutations observed on the minus strand (and vice versa), four mutations were identified (Data now shown). The number of SDMs was taken to be the minimum of the number of supermutants on the plus or the minus strand that corresponded to a mutation, as this is the limiting number of double stranded supermutant molecules detectable. The total number of double stranded molecules was similarly taken to be the minimum of the number of total UIDs on the plus or the minus strand, as this is the limiting number of double stranded template molecules detected. Standard NGS detected 197 and 167 indels on the plus and minus strands, respectively. Use of molecular barcodes reduced the number of detected indels to 6 and 5 for the plus and minus strand, respectively, while BiSeqS double strand analysis reduced the number of indels to zero.

### EXAMPLE 2

### BiSeqS Workflow

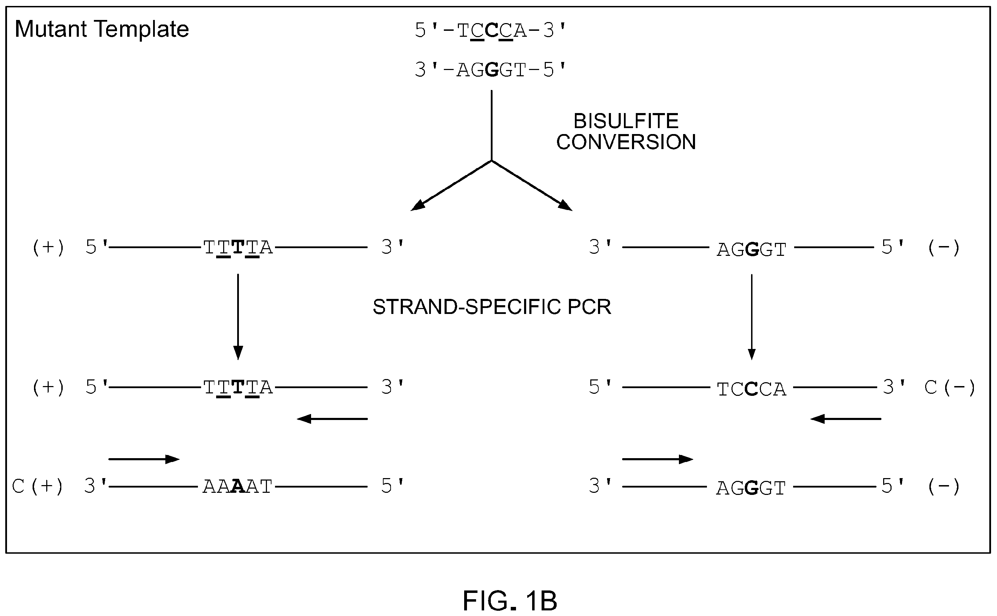

The principal feature of BiSeqS is the simultaneous detection of a mutation on both the plus and minus strands of DNA templates that were bisulfite treated and molecularly barcoded. We refer to the reference sequence as defined by UCSC as the plus (+) strand, and its reverse complement as the minus (-) strand. Three simple experimental steps (bisulfite conversion, molecular barcoding, and sample barcoding) can be employed prior to a specialized bioinformatics analysis of the sequencing data, as described below (Fig. 1 and Fig. 4A-B).

**Step i: Bisulfite Conversion.** Incubation of DNA with sodium bisulfite at elevated temperatures and low pH deaminates cytosine to form 5,6-dihydrocytosine-6-sulfonate (34). Subsequent hydrolytic deamination at high pH removes the sulfonate, resulting in uracil (35). Many modifications of this basic reaction have been described and used largely to differentiate between cytosine and 5-methylcytosine (5-mC), the latter of which is not susceptible to bisulfite conversion. In addition to converting C to U, bisulfite treatment denatures DNA and can degrade it. Although this degradation is not limiting for standard applications of bisulfite treatment, it is critical for applications involving mutation detection in clinical samples that are already degraded prior to conversion (36-38). In the current study, we evaluated many ways to convert DNA, and purify the converted strands. The best results were obtained with the reagents, conditions, and incubation times described in the Materials and Methods. As shown in Fig. 5, treatment under these conditions did not inhibit the amplification of PCR products up to 285 bp in size. Sequencing of these products revealed that, on average, > 99.8% of the C bases were converted to T bases on both strands (excluding C bases at 5'-CpG sites, which can be resistant to bisulfite conversion because they are either methylated or hydroxymethylated).

**Step ii. Molecular Barcoding.** The goal of bisulfite treatment is to create a code for distinguishing the two strands of DNA. This doubles the number of templates that need to be molecularly barcoded, utilizing specialized steps compared to that used for standardly amplifying DNA. First, four primers must be designed to amplify each region of interest, two primers for each strand. Second, the primers must be complimentary to the converted form of the DNA, accentuating the importance of full conversion - otherwise, some template molecules will not be amplified because they will not be perfectly complementary to the primers. Third, bisulfite treatment under the conditions we employed converts virtually all non-modified C residues to T, lowering the melting temperature of both the primer annealing sites and the amplicon in general. Because both strands must be amplified equivalently and in the same reaction, the primers must be chosen so that the same PCR cycling conditions can be used for amplifying both strands in a highly specific manner. For regions in which there is already a low C:G base pair content, the primers have to be long enough to allow specific amplification under relatively high-temperature annealing conditions. This proved difficult without yielding large amounts of primer dimers, and to overcome these challenges, several primer designs were evaluated. Eventually, variations in primer length, position, composition and C:G content allowed for specific and robust amplification of both strands of every target region attempted.

Another issue confronting amplification of bisulfite converted DNA is that many polymerases will not efficiently copy DNA that contains uracil bases. We tested seven commercially available polymerases and various reaction conditions to optimize efficiency of template use and uniformity of amplification of both strands when four primers were used (Table 1). While a combination of AMPIGene Hot Start Taq Polymerase and iTAQ Polymerase amplified the greatest number of template molecules, their lack of 3' → 5' exonuclease activity proved limiting for specificity in that the number of errors during PCR was unacceptably high. Ultimately, we chose Phusion U Hot Start Polymerase, a polymerase that exhibits 3' → 5' exonuclease activity, as the enzyme to amplify uracil-containing templates with the highest specificity while maintaining sensitivity.

**Step iii: Sample Barcoding.** Part of the power of massively parallel sequencing instruments is that they can be used to analyze many samples at once. To enable this capacity for BiSeqS, we incorporated a sample barcode PCR cycle following the purification of the molecularly barcoded PCR products (Fig. 4, step iii). Moreover, the converted sample DNA was divided into two to six wells of the PCR plate prior to the molecular barcoding step. Each well was then assigned a different sample barcode. This distribution served two purposes. First, with concentrated DNA templates, it could provide independent replication of mutations with small mutant allele fractions. Second, with dilute DNA templates, as are often present in clinical samples such as plasma (9), urine (39), and CSF (12), it provides the opportunity to test more template molecules, increasing the chance of identifying mutant templates.

### EXAMPLE 3

### BiSeqS Data Processing Pipeline

High quality base calls were aligned to the bisulfite-converted reference sequence, and the aligned data were organized into tables for each sample, where each observed mutation in each strand of each well was listed in a separate row. The columns in this table included the number of reads, UIDs, and supermutants for each mutation (data not shown). Supermutants were defined as mutations in a UID family in which > 90% of the family members contained that mutation. For example, if all three members of a UID family contained the same mutation, it was considered a supermutant. The supermutant allele fraction was defined as the number of supermutants divided by the number of UIDs in an individual well.

Individual mutations in the plus and minus strands were compared to determine whether the identical supermutant was found in both strands. If the mutation was found in both strands, the supermutant allele fractions in each strand were compared. The supermutant allele fractions on each strand provide an additional level of specificity because these fractions are expected to be similar if a mutant base pair existed in the template DNA prior to conversion and amplification. Given that mutations arising during PCR are relatively rare, it would be even rarer for the same mutation to arise at the identical position in both strands. This is especially true after conversion, when the two strands contain markedly different nucleotide contexts. If the supermutant allele fractions in each strand differed by < 10-fold, then the mutation was considered to be a super-duper mutant (SDM). The SDM allelic fraction was defined as the number of SDMs divided by the number of UIDs in the strand that contained the fewest UIDs. For example, if the number of SDMs was 10, and the number of UIDs in the plus and minus strands were 10,000 and 20,000, respectively, then the SDM allelic fraction would be 0.1% (i.e., 10 of 10,000).

Special features of the analysis of mutations in converted DNA include the following. A transition from C >T noted in the sequencing could have resulted from a single base substitution mutation that changed a C:G bp to a T:A bp or from bisulfite conversion of a C to a T on one strand. In light of this ambiguity, C to T mutations cannot be considered supermutants in the strand containing the C, though a supermutant would still be evident at that position in the strand containing the G. There are a total of six possible single base substitutions in duplex DNA: A C:G bp can be mutated to either A:T, G:C, or T:A bps, and an A:T bp can be mutated to either C:G: G:C, or T:A. Of these six single base pair substitutions, all result in supermutants on at least one strand and four result in supermutants on both strands (*i.e.,* SDMs). In addition, transitions that create a CpG dinucleotide in which the C is methylated can be assessed on both strands. All insertions or deletions within the amplified sequences can form SDMs. Methylation also introduces complexity, as methylated or hydroxymethylated C bases are not converted to U bases by bisulfite treatment. The BiSeqS pipeline takes this into account when it analyzes the data by not assuming that any particular C is methylated or unmethylated (or that every unmethylated C is converted to T by bisulfite treatment). Instead, it considers the possible effects of conversion and methylation and only labels a mutation as a supermutant or SDM if there is no ambiguity. A list of all possible single base substitutions on either strand, within a triplet context and with the mutated base in the middle, is provided in Table 1, below.

| **Triplet From** | **Triplet To** | **Scorable Strands** | **Does Mutation Create New CpG Site?** |
|---|---|---|---|
| AAG | ACG | BOTH | YES |
| AGG | ACG | BOTH | YES |
| ATG | ACG | BOTH | YES |
| CAG | CCG | BOTH | YES |
| CCA | CGA | BOTH | YES |
| CCC | CGC | BOTH | YES |
| CCG | CGG | BOTH | YES |
| CCT | CGT | BOTH | YES |
| CGG | CCG | BOTH | YES |
| CTA | CGA | BOTH | YES |
| CTC | CGC | BOTH | YES |
| CTG | CCG | BOTH | YES |
| CTT | CGT | BOTH | YES |
| GAG | GCG | BOTH | YES |
| GGG | GCG | BOTH | YES |
| TAG | TCG | BOTH | YES |
| TGG | TCG | BOTH | YES |
| TTG | TCG | BOTH | YES |
| AAA | ACA | BOTH | NO |
| AAA | ATA | BOTH | NO |
| AAC | ACC | BOTH | NO |
| AAC | ATC | BOTH | NO |
| AAG | ATG | BOTH | NO |
| AAT | ACT | BOTH | NO |
| AAT | ATT | BOTH | NO |
| ACA | AAA | BOTH | NO |
| ACA | AGA | BOTH | NO |
| ACC | AAC | BOTH | NO |
| ACC | AGC | BOTH | NO |
| ACG | AAG | BOTH | NO |
| ACG | AGG | BOTH | NO |
| ACT | AAT | BOTH | NO |
| ACT | AGT | BOTH | NO |
| AGA | ACA | BOTH | NO |
| AGA | ATA | BOTH | NO |
| AGC | ACC | BOTH | NO |
| AGC | ATC | BOTH | NO |
| AGG | ATG | BOTH | NO |
| AGT | ACT | BOTH | NO |
| AGT | ATT | BOTH | NO |
| ATA | AAA | BOTH | NO |
| ATA | AGA | BOTH | NO |
| ATC | AAC | BOTH | NO |
| ATC | AGC | BOTH | NO |
| ATG | AAG | BOTH | NO |
| ATG | AGG | BOTH | NO |
| ATT | AAT | BOTH | NO |
| ATT | AGT | BOTH | NO |
| CAC | CCA | BOTH | NO |
| CAC | CTA | BOTH | NO |
| CAC | CCC | BOTH | NO |
| CAC | CTC | BOTH | NO |
| CAG | CTG | BOTH | NO |
| CAT | CCT | BOTH | NO |
| CAT | CTT | BOTH | NO |
| CCA | CAA | BOTH | NO |
| CCC | CAC | BOTH | NO |
| CCG | CAG | BOTH | NO |
| CCT | CAT | BOTH | NO |
| CGA | CCA | BOTH | NO |
| CGA | CTA | BOTH | NO |
| CGC | CCC | BOTH | NO |
| CGC | CTC | BOTH | NO |
| CGG | CTG | BOTH | NO |
| CGT | CCT | BOTH | NO |
| CGT | CTT | BOTH | NO |
| CTA | CAA | BOTH | NO |
| CTC | CAC | BOTH | NO |
| CTG | CAG | BOTH | NO |
| CTG | CGG | BOTH | NO |
| CTT | CAT | BOTH | NO |
| GAA | GCA | BOTH | NO |
| GAA | GTA | BOTH | NO |
| GAC | GCC | BOTH | NO |
| GAC | GTC | BOTH | NO |
| GAG | GTG | BOTH | NO |
| GAT | GCT | BOTH | NO |
| GAT | GTT | BOTH | NO |
| GCA | GAA | BOTH | NO |
| GCA | GGA | BOTH | NO |
| GCC | GAC | BOTH | NO |
| GCC | GGC | BOTH | NO |
| GCG | GAG | BOTH | NO |
| GCG | GGG | BOTH | NO |
| GCT | GAT | BOTH | NO |
| GCT | GGT | BOTH | NO |
| GGA | GCA | BOTH | NO |
| GGA | GTA | BOTH | NO |
| GGC | GCC | BOTH | NO |
| GGC | GTC | BOTH | NO |
| GGG | GTG | BOTH | NO |
| GGT | GCT | BOTH | NO |
| GGT | GTT | BOTH | NO |
| GTA | GAA | BOTH | NO |
| GTA | GGA | BOTH | NO |
| GTC | GAC | BOTH | NO |
| GTC | GGC | BOTH | NO |
| GTG | GAG | BOTH | NO |
| GTG | GGG | BOTH | NO |
| GTT | GAT | BOTH | NO |
| GTT | GGT | BOTH | NO |
| TAA | TCA | BOTH | NO |
| TAA | TTA | BOTH | NO |
| TAC | TCC | BOTH | NO |
| TAC | TTC | BOTH | NO |
| TAG | TTG | BOTH | NO |
| TAT | TCT | BOTH | NO |
| TAT | TTT | BOTH | NO |
| TCA | TAA | BOTH | NO |
| TCA | TGA | BOTH | NO |
| TCC | TAC | BOTH | NO |
| TCC | TGC | BOTH | NO |
| TCG | TAG | BOTH | NO |
| TCG | TGG | BOTH | NO |
| TCT | TAT | BOTH | NO |
| TCT | TGT | BOTH | NO |
| TGA | TCA | BOTH | NO |
| TGA | TTA | BOTH | NO |
| TGC | TCC | BOTH | NO |
| TGC | TTC | BOTH | NO |
| TGG | TTG | BOTH | NO |
| TGT | TCT | BOTH | NO |
| TGT | TTT | BOTH | NO |
| TTA | TAA | BOTH | NO |
| TTA | TGA | BOTH | NO |
| TTC | TAC | BOTH | NO |
| TTC | TGC | BOTH | NO |
| TTG | TAG | BOTH | NO |
| TTG | TGG | BOTH | NO |
| TTT | TAT | BOTH | NO |
| TTT | TGT | BOTH | NO |
| AAA | AGA | (+) STRAND | NO |
| AAC | AGC | (+) STRAND | NO |
| AAG | AGG | (+) STRAND | NO |
| AAT | AGT | (+) STRAND | NO |
| AGA | AAA | (+) STRAND | NO |
| AGC | AAC | (+) STRAND | NO |
| AGG | AAG | (+) STRAND | NO |
| CAC | CGA | (+) STRAND | NO |
| CAC | CGC | (+) STRAND | NO |
| CAG | CGG | (+) STRAND | NO |
| CAT | CGT | (+) STRAND | NO |
| CGA | CAA | (+) STRAND | NO |
| CGC | CAC | (+) STRAND | NO |
| CGG | CAG | (+) STRAND | NO |
| CGT | CAT | (+) STRAND | NO |
| GAA | GGA | (+) STRAND | NO |
| GAC | GGC | (+) STRAND | NO |
| GAG | GGG | (+) STRAND | NO |
| GAT | GGT | (+) STRAND | NO |
| GGA | GAA | (+) STRAND | NO |
| GGC | GAC | (+) STRAND | NO |
| GGG | GAG | (+) STRAND | NO |
| GGT | GAT | (+) STRAND | NO |
| TAA | TGA | (+) STRAND | NO |
| TAC | TGC | (+) STRAND | NO |
| TAG | TGG | (+) STRAND | NO |
| TAT | TGT | (+) STRAND | NO |
| TGA | TAA | (+) STRAND | NO |
| TGC | TAC | (+) STRAND | NO |
| TGG | TAG | (+) STRAND | NO |
| TGT | TAT | (+) STRAND | NO |
| ACA | ATA | (-) STRAND | NO |
| ACC | ATC | (-) STRAND | NO |
| ACG | ATG | (-) STRAND | NO |
| ACT | ATT | (-) STRAND | NO |
| AGT | AAT | (-) STRAND | NO |
| ATA | ACA | (-) STRAND | NO |
| ATC | ACC | (-) STRAND | NO |
| ATT | ACT | (-) STRAND | NO |
| CCA | CTA | (-) STRAND | NO |
| CCC | CTC | (-) STRAND | NO |
| CCG | CTG | (-) STRAND | NO |
| CCT | CTT | (-) STRAND | NO |
| CTA | CCA | (-) STRAND | NO |
| CTC | CCC | (-) STRAND | NO |
| CTT | CCT | (-) STRAND | NO |
| GCA | GTA | (-) STRAND | NO |
| GCC | GTC | (-) STRAND | NO |
| GCG | GTG | (-) STRAND | NO |
| GCT | GTT | (-) STRAND | NO |
| GTA | GCA | (-) STRAND | NO |
| GTC | GCC | (-) STRAND | NO |
| GTG | GCG | (-) STRAND | NO |
| GTT | GCT | (-) STRAND | NO |
| TCA | TTA | (-) STRAND | NO |
| TCC | TTC | (-) STRAND | NO |
| TCG | TTG | (-) STRAND | NO |
| TCT | TTT | (-) STRAND | NO |
| TTA | TCA | (-) STRAND | NO |
| TTC | TCC | (-) STRAND | NO |
| TTT | TCT | (-) STRAND | NO |

For each single base substitution, the capacity of BiSeqS to identify SDMs is also provided in this table. In general terms, all transversions, all insertions and deletions, and a small subset of transitions can be unambiguously scored as SDMs (Table 1). Because the power of BiSeqS lies in SDMs, only mutations that are interpretable in both strands are considered below.

### EXAMPLE 4

### BiSeqS Increases the Specificity of Mutation Calling

We selected eight amplicons within prototypic cancer driver genes to assess BiSeqS performance. For each of the eight amplicons, two forward primers and two reverse primers for each strand were synthesized and tested using the principles described above and in the Materials and Methods. For all amplicons, at least one primer pair for each strand was found capable of specifically amplifying the intended strand with high efficiency, as judged by polyacrylamide gel analysis (Fig. 5). The sequences of these primers are listed in the sequence listing.

For each of the eight amplicons, we compared the specificity of BiSeqS to that of conventional next generation sequencing (NGS) and molecular barcode-assisted sequencing (*i.e.,* SafeSeqS). We considered only those potential mutations that could be discerned in both strands, as described above. There were a total of 608 bp within these amplicons, yielding a total of 1550 single base substitutions possible. Of these 1550 potential SBS, 1252 (80.8%) were scorable as SDMs; the remainder were transitions that were not scorable for the reasons noted above. There were also many possible indels at each position that could have been observed in the sequencing data, all scorable as SDMs.

In the actual experiment, we could distinguish the strand used as template in the sequencing instrument because of the bisulfite conversion. In light of this, there were actually 2504 mutations (2 x the number of bp) that could be scored for both conventional and molecular-barcode assisted sequencing. Of these 2504 potential SBSs, 1865 (74.5% of the total possible mutations) were actually observed upon conventional sequencing (25), highlighting the relatively large number of errors observed unless error correction by SafeSeqS or BiSeqS is applied (data not shown). There was no discernible difference between the two strands with respect to the number of mutations observed, with 907 and 958 mutations observed on the plus and minus strands, respectively. There were also 298 small insertions or deletions observed by conventional NGS.

Application of the molecular barcoding approach to these data considerably reduced the number of mutations, as evident by comparison of Fig. 6A and 6B (note that the y-axis scale was reduced by two orders of magnitude in Fig. 6B). The most relevant measure of this reduction is the comparison of the mutant allele frequencies (MAFs) before and after molecular barcoding was applied. Before molecular barcoding was applied, the median mutant allele frequencies (MAFs) of the SBS in the plus strand was 0.0233% (average 0.0720%, 95% CI 0.0627% to 0.0813%; Fig. 2A-C). It was similar in the minus strand: median of 0.0185%, average of 0.0751%, 95% CI 0.0643% to 0.0859%. As shown in Fig. 2B, after molecular barcoding, the MAF in the plus strand was reduced by 8-fold, to a median of 0.0000%, average of 0.0091% (95% CI of 0.0062% to 0.0119%; p <10-12, paired two-tailed student's t-test). Note that the MAF after molecular barcoding is a measure of supermutant allele frequency (SMAF), but is labeled MAF in Fig. 2B for simplicity. The MAF of the minus strand was reduced by 9-fold by molecular barcoding (median of 0.0000%, average of 0.0080%, 95% CI of 0.0047% to 0.0113%; p <10-12, paired two-tailed student's t-test). The magnitude of the reductions achieved by SafeSeqS were in accordance with expectations from experiments on native DNA that had not been treated with bisulfite (27).

Application of BiSeqS to these data resulted in a further striking reduction in errors. Only four SDMs were observed over all eight amplicons sequenced, as opposed to 1865 and 163 mutations without and with molecular barcoding, respectively (Fig. 6; note that y-axis of Fig. 6C has been reduced by another order of magnitude compared to Fig. 6B). This was reflected in the MAFs, as shown in Fig. 2C, which were reduced by 1217-fold through BiSeqS compared to NGS and 141-fold compared to molecular barcoding (median of 0.0000%, average of 0.0001%, 95% CI of 0.0000% to 0.0001%; p <10-12, paired two-tailed student's t-test).

BiSeqS also reduced errors at indels; there were 364 mutants, 11 supermutants, and zero SDMs observed in the eight amplicons (Fig. 7 and 8). The MAFs were thereby reduced from an average of 0.0041% with NGS to 0.0011% with molecular barcoding to 0.0000% with BiSeqS (p < 1.2 x 10⁻⁶ for NGS compared to molecular barcoding for the plus strand, p < 7.5 x 10⁻⁴ for NGS compared to molecular barcoding for the minus strand, p < 1.3 x 10⁻² for molecular barcoding compared to BiSeqS).

### EXAMPLE 5

### Sensitivity of BiSeqS

Massively parallel sequencing allows billions of amplicons to be assessed simultaneously, resulting in theoretical sensitivities of 1 mutation among > 1 billion WT templates for any base within an amplicon. The actual sensitivities in clinical samples are limited only by the amount of input DNA and the specificity. In many types of liquid biopsies, such as those from plasma, pancreatic cysts, CSF, and urine, the total DNA available is often < 33 ng (7, 9, 12, 39). A sensitivity of 0.01% is therefore adequate for detecting the one or two mutant molecules that may exist among the ~10,000 templates contained in 33 ng of human DNA in such samples. The reliability of this detection is limited by the biological and technical specificities, where the queried mutation must be found at far lower frequencies in the normal control samples used for comparison to the tumor. Although the biological issues that might lead to mutations in normal samples cannot be circumvented (40), technical issues can be addressed and overcome through methodological advances such as BiSeqS.

To address the sensitivity of BiSeqS, we evaluated tumor samples containing ten double-stranded mutations (20 mutations if each strand is counted separately) within the eight amplicons described above (data not shown). The proportion of mutations in each of the tumor samples was defined through NGS. We used the DNA from these tumors to create the scenario characteristic of liquid biopsies, wherein a small amount of DNA from neoplastic cells is mixed with a much larger amount of DNA from normal cells in the patient. More specifically, we diluted this tumor DNA with normal leukocytes to achieve minor allele fractions of 0.02% and 0.20% and then used bisulfite treatment to convert the mixtures. We determined the mutant allele fractions of each of the tumor-derived mutations when analyzed with standard NGS, with molecular barcodes, or with BiSeqS, in all cases holding the input DNA to 5,000 template molecules per well, and performing each experiment in six wells. We found that each of the three methods of analysis yielded mutant allele fractions that were similar to those expected from the dilutions (examples in Fig. 3). This experiment demonstrated that the efficiency of each of the steps in BiSeqS - from bisulfite conversion through the amplification and sequencing steps - was high.

Although the efficiency of amplification was therefore always high enough to detect the mutant templates, the MAFs of the normal controls limited the interpretation of the sequencing data. We called a mutant call a true mutation when the signal-to-noise ratios (SNRs), defined as the MAF in the tumor specimen divided by the MAF in normal cells, was >10. We averaged the MAF in both strands for this calculation when considering standard NGS or molecular barcode-assisted NGS. Fig. 3 and Fig. 9 show the detected MAFs for dilutions of 0.20% and 0.02%. Standard NGS yielded SNRs >10 for only two of the eight mutations at a neoplastic cell content of 0.20% and one out of the three mutations at neoplastic cell contents of 0.02%. Molecular barcoding yielded SNRs >10 for 7 of the 10 mutations at these neoplastic cell contents. In contrast, BiSeqS yielded SNR >10 for all 10 mutations at all tested neoplastic cell fractions (Fig. 3, Fig. 9). Representative SNR plots of the MAF for mutations in NRAS and TP53 are shown in Fig. 10A and 10B, respectively.

### EXAMPLE 6

### BiSeqS Simultaneously Detects Methylation Status on Both Strands

Cytosine bases in 5'-CpG dinucleotides that are methylated are protected from conversion to uracil during bisulfite treatment, allowing BiSeqS to detect the methylation status of the plus and minus strands simultaneously. Although not the primary purpose of BiSeqS, this discrimination could prove useful for the analysis of methylation that occurs at low levels, either for basic research or clinical purposes. Although bisulfite treatment and specially-designed primers have often been used to evaluate methylation in the past for a variety of clinical purposes (41-43), the combination of molecular barcoding with simultaneous amplification of both strands provides unprecedented sensitivity in this type of analysis.

To demonstrate the ability of BiSeqS to discriminate the methylation status on both strands simultaneously, we evaluated a region of the TP53 gene that contains a known methylated CpG at hg19 position 7,572,973 to 4. Greater than 90% of the UIDs on both strands were found to be methylated at the C at the plus strand of position 7,572,973 and the C opposite the G on the minus strand at position 7,572,974. Greater than 99.8% of the C residues that were not at 5'-CpG dinucleotides within this amplicon were found to be converted to T's, providing an essential control for interpreting the extent of methylation. We then searched for evidence of double-stranded methylation within all eight amplicons evaluated in this study in normal WBCs. There were two 5'-CpG residues within the 608 bp that could be evaluated. Of these, we found that both CpG's were methylated on both strands, with the fraction of methylated alleles ranging from 92.10% to 96.10% (data not shown).

Sequence Listing: The SEQ ID NOs are indicated by field <210>, and the sequence is indicated by field <400>
<110> The Johns Hopkins University
<120> STRAND-SPECIFIC DETECTION OF
   BISULFITE-CONVERTED DUPLEXES
<130> 44807-0125WO1
<150> 62/476,234
   <151> 2017-03-24
<160> 48
<170> FastSEQ for Windows Version 4.0
<210> 1
   <211> 65
   <212> DNA
   <213> Artificial Sequence
<220>
   <223> Chemically modified sequences
<221> misc_feature
   <222> (1)...(65)
   <223> n = A,T,C or G
<400> 1
   cgacgtaaaa cgacggccag tnnnnnnnnn nnnnnggatt agttggattg ttagtgtgtt 60
   ttttt 65
<210> 2
   <211> 77
   <212> DNA
   <213> Artificial Sequence
<220>
   <223> Chemically modified sequences
<221> misc_feature
   <222> (1)...(77)
   <223> n = A,T,C or G
<400> 2
   cgacgtaaaa cgacggccag tnnnnnnnnn nnnnncaaaa taattctaaa ttaactaaat 60
   tatcaataca cttttcc 77
<210> 3
   <211> 78
   <212> DNA
   <213> Artificial Sequence
<220>
   <223> Chemically modified sequences
<221> misc_feature
   <222> (1)...(78)
   <223> n = A,T,C or G
<400> 3
   cgacgtaaaa cgacggccag tnnnnnnnnn nnnnnagtat tgagaaattg ataaatgtat 60
   gaatagtatt aaattaga 78
<210> 4
   <211> 73
   <212> DNA
   <213> Artificial Sequence
<220>
   <223> Chemically modified sequences
<221> misc_feature
   <222> (1)...(73)
   <223> n = A,T,C or G
<400> 4
   cgacgtaaaa cgacggccag tnnnnnnnnn nnnnncaaaa aattaacaaa catataaaca 60
   acattaaacc aaa 73
<210> 5
   <211> 83
   <212> DNA
   <213> Artificial Sequence
<220>
   <223> Chemically modified sequences
<221> misc_feature
   <222> (1)...(83)
   <223> n = A,T,C or G
<400> 5
   cgacgtaaaa cgacggccag tnnnnnnnnn nnnnnatttg ttttgatgta ataaatatgt 60
   atatattatt atattagttt gtt 83
<210> 6
   <211> 76
   <212> DNA
   <213> Artificial Sequence
<220>
   <223> Chemically modified sequences
<221> misc_feature
   <222> (1)...(76)
   <223> n = A,T,C or G
<400> 6
   cgacgtaaaa cgacggccag tnnnnnnnnn nnnnnccaat ataataaata tacacatatc 60
   attacaccaa ttcatc 76
<210> 7
   <211> 71
   <212> DNA
   <213> Artificial Sequence
<220>
   <223> Chemically modified sequences
<221> misc_feature
   <222> (1)...(71)
   <223> n = A,T,C or G
<400> 7
   cgacgtaaaa cgacggccag tnnnnnnnnn nnnnngggaa tttaaagtat atgaatttgt 60
   ttttttgttg t 71
<210> 8
   <211> 67
   <212> DNA
   <213> Artificial Sequence
<220>
   <223> Chemically modified sequences
<221> misc_feature
   <222> (1)...(67)
   <223> n = A,T,C or G
<400> 8
   cgacgtaaaa cgacggccag tnnnnnnnnn nnnnnactca aaatacataa acttatcttc 60
   ccatcat 67
<210> 9
   <211> 74
   <212> DNA
   <213> Artificial Sequence
<220>
   <223> Chemically modified sequences
<221> misc_feature
   <222> (1)...(74)
   <223> n = A,T,C or G
<400> 9
   cgacgtaaaa cgacggccag tnnnnnnnnn nnnnngaaga taagtttatg tattttgagt 60
   ttttttagtt gtta 74
<210> 10
   <211> 69
   <212> DNA
   <213> Artificial Sequence
<220>
   <223> Chemically modified sequences
<221> misc_feature
   <222> (1)...(69)
   <223> n = A,T,C or G
<400> 10
   cgacgtaaaa cgacggccag tnnnnnnnnn nnnnncaaat tcatatactt taaattccct 60
   caaccatta 69
<210> 11
   <211> 64
   <212> DNA
   <213> Artificial Sequence
<220>
   <223> Chemically modified sequences
<221> misc_feature
   <222> (1)...(64)
   <223> n = A,T,C or G
<400> 11
   cgacgtaaaa cgacggccag tnnnnnnnnn nnnnnggaga aaagtattgg ttggttttgt 60
   tttt 64
<210> 12
   <211> 80
   <212> DNA
   <213> Artificial Sequence
<220>
   <223> Chemically modified sequences
<221> misc_feature
   <222> (1)...(80)
   <223> n = A,T,C or G
<400> 12
   cgacgtaaaa cgacggccag tnnnnnnnnn nnnnnactaa ccttaaaatt taaaaaaaaa 60
   tatcaattaa ctttatcttt 80
<210> 13
   <211> 56
   <212> DNA
   <213> Artificial Sequence
<220>
   <223> Chemically modified sequences
<221> misc_feature
   <222> (1)...(56)
   <223> n = A,T,C or G
<400> 13
   cgacgtaaaa cgacggccag tnnnnnnnnn nnnnngtgtg tagggtgaag tgtgag 56
<210> 14
   <211> 80
   <212> DNA
   <213> Artificial Sequence
<220>
   <223> Chemically modified sequences
<221> misc_feature
   <222> (1)...(80)
   <223> n = A,T,C or G
<400> 14
   cgacgtaaaa cgacggccag tnnnnnnnnn nnnnnccaaa actaaataaa actccaaata 60
   tatataaaac aaaatataaa 80
<210> 15
   <211> 66
   <212> DNA
   <213> Artificial Sequence
<220>
   <223> Chemically modified sequences
<221> misc_feature
   <222> (1)...(66)
   <223> n = A,T,C or G
<400> 15
   cgacgtaaaa cgacggccag tnnnnnnnnn nnnnnggaga atgttagttt gagttaggtt 60
   tttttg 66
<210> 16
   <211> 74
   <212> DNA
   <213> Artificial Sequence
<220>
   <223> Chemically modified sequences
<221> misc_feature
   <222> (1)...(74)
   <223> n = A,T,C or G
<400> 16
   cgacgtaaaa cgacggccag tnnnnnnnnn nnnnncaaaa aataaaaaat atcaatctaa 60
   atcaaaccct tcta 74
<210> 17
   <211> 71
   <212> DNA
   <213> Artificial Sequence
<220>
   <223> Chemically modified sequences
<400> 17
   cacacaggaa acagctatga ccatgattat aaaaaacttt aaaatactat aaatataact 60
   caccaattaa c 71
<210> 18
   <211> 62
   <212> DNA
   <213> Artificial Sequence
<220>
   <223> Chemically modified sequences
<400> 18
   cacacaggaa acagctatga ccatgagttt taaagtattg tagatgtggt ttgttaatta 60
   at 62
<210> 19
   <211> 58
   <212> DNA
   <213> Artificial Sequence
<220>
   <223> Chemically modified sequences
<400> 19
   cacacaggaa acagctatga ccatgcatta actcatccta aattataaca atcaccaa 58
<210> 20
   <211> 70
   <212> DNA
   <213> Artificial Sequence
<220>
   <223> Chemically modified sequences
<400> 20
   cacacaggaa acagctatga ccatggaaaa gagaatgtat taatttattt tgaattgtag 60
   taattattaa 70
<210> 21
   <211> 63
   <212> DNA
   <213> Artificial Sequence
<220>
   <223> Chemically modified sequences
<400> 21
   cacacaggaa acagctatga ccatgcaact aaaacttatc aaaccctttt ataaaaatct 60
   taa 63
<210> 22
   <211> 69
   <212> DNA
   <213> Artificial Sequence
<220>
   <223> Chemically modified sequences
<400> 22
   cacacaggaa acagctatga ccatgttatt atagttagaa tttattaaat ttttttgtga 60
   agattttga 69
<210> 23
   <211> 70
   <212> DNA
   <213> Artificial Sequence
<220>
   <223> Chemically modified sequences
<400> 23
   cacacaggaa acagctatga ccatgtttaa caatttaaca attaaaaaca tttcctataa 60
   aataatacta 70
<210> 24
   <211> 64
   <212> DNA
   <213> Artificial Sequence
<220>
   <223> Chemically modified sequences
<400> 24
   cacacaggaa acagctatga ccatgagttt gatagttaaa ggtatttttt gtgaaataat 60
   attg 64
<210> 25
   <211> 66
   <212> DNA
   <213> Artificial Sequence
<220>
   <223> Chemically modified sequences
<400> 25
   cacacaggaa acagctatga ccatgtttta atctatcctt attttaaata tttctcccaa 60
   taaaaa 66
<210> 26
   <211> 69
   <212> DNA
   <213> Artificial Sequence
<220>
   <223> Chemically modified sequences
<400> 26
   cacacaggaa acagctatga ccatgagttt ttaatttgtt tttattttgg atattttttt 60
   taatgaaag 69
<210> 27
   <211> 73
   <212> DNA
   <213> Artificial Sequence
<220>
   <223> Chemically modified sequences
<400> 27
   cacacaggaa acagctatga ccatgcaaaa aaaataaaaa ataaaaatct atataatcaa 60
   aaaatcaata aca 73
<210> 28
   <211> 60
   <212> DNA
   <213> Artificial Sequence
<220>
   <223> Chemically modified sequences
<400> 28
   cacacaggaa acagctatga ccatgaaatg gaagtttatg tgattaagaa attgatagta 60
<210> 29
   <211> 60
   <212> DNA
   <213> Artificial Sequence
<220>
   <223> Chemically modified sequences
<400> 29
   cacacaggaa acagctatga ccatgccact atacctatac aataccaata ataacaacaa 60
<210> 30
   <211> 58
   <212> DNA
   <213> Artificial Sequence
<220>
   <223> Chemically modified sequences
<400> 30
   cacacaggaa acagctatga ccatggttgt atttgtgtaa tgttagtgat gatgataa 58
<210> 31
   <211> 54
   <212> DNA
   <213> Artificial Sequence
<220>
   <223> Chemically modified sequences
<400> 31
   cacacaggaa acagctatga ccatgctcat ataatatcat ctctcctccc tact 54
<210> 32
   <211> 69
   <212> DNA
   <213> Artificial Sequence
<220>
   <223> Chemically modified sequences
<400> 32
   cacacaggaa acagctatga ccatggtata gattttttta tttatgtgat gttatttttt 60
   ttttttgtt 69
<210> 33
   <211> 158
   <212> DNA
   <213> Artificial Sequence
<220>
   <223> Chemically modified sequences
<400> 33
   ggattagttg gattgttagt gtgttttttt taatattatt tgttttaatt attattagtt 60
   tgtatttagt tattttatat tagtaagaat ttgttggaaa ttagtaatta gggttaattg 120
   gtgagttata tttatagtat tttaaagttt tttataat 158
<210> 34
   <211> 161
   <212> DNA
   <213> Artificial Sequence
<220>
   <223> Chemically modified sequences
<400> 34
   caaaataatt ctaaattaac taaattatca atacactttt cccaacacca cctactccaa 60
   ccaccaccaa tttatactca atcatttcac accaacaaaa acctattaaa aaccaataat 120
   caaaattaat taacaaacca catctacaat actttaaaac t 161
<210> 35
   <211> 150
   <212> DNA
   <213> Artificial Sequence
<220>
   <223> Chemically modified sequences
<400> 35
   agtattgaga aattgataaa tgtatgaata gtattaaatt agattttatt tagttgagaa 60
   agatgagaga ttaatatttg atgtaagtat ttgaaatgga attttatata tgaataattg 120
   gtgattgtta taatttagga tgagttaatg 150
<210> 36
   <211> 157
   <212> DNA
   <213> Artificial Sequence
<220>
   <223> Chemically modified sequences
<400> 36
   caaaaaatta acaaacatat aaacaacatt aaaccaaacc ttatccaact aaaaaaaaca 60
   aaaaaccaat acttaatata aatatttaaa ataaaatcct atacataaat aattaataat 120
   tactacaatt caaaataaat taatacattc tcttttc 157
<210> 37
   <211> 145
   <212> DNA
   <213> Artificial Sequence
<220>
   <223> Chemically modified sequences
<400> 37
   atttgttttg atgtaataaa tatgtatata ttattatatt agtttgtttt tttttagttt 60
   tatagtgaat tgttgtaata tgattgttat ttttatttag ttattggtta agatttttat 120
   aaaagggttt gataagtttt agttg 145
<210> 38
   <211> 144
   <212> DNA
   <213> Artificial Sequence
<220>
   <223> Chemically modified sequences
<400> 38
   ccaatataat aaatatacac atatcattac accaattcat ccctttccaa ctttacaata 60
   aattactaca acataattat catcttcact taaccattaa tcaaaatctt cacaaaaaaa 120
   tttaataaat tctaactata ataa 144
<210> 39
   <211> 162
   <212> DNA
   <213> Artificial Sequence
<220>
   <223> Chemically modified sequences
<400> 39
   gggaatttaa agtatatgaa tttgtttttt tgttgtgtgg gttttgaatt ggaggaatat 60
   atttttattt ttagttggta gattataaat tgaggatttg tatggttaaa tatatattag 120
   tattatttta taggaaatgt ttttaattgt taaattgtta aa 162
<210> 40
   <211> 152
   <212> DNA
   <213> Artificial Sequence
<220>
   <223> Chemically modified sequences
<400> 40
   actcaaaata cataaactta tcttcccatc atataaatcc taaattaaaa aaatatatct 60
   tcacctttaa ctaacaaacc acaaactaaa aatctacata attaaataca taccaatatt 120
   atttcacaaa aaataccttt aactatcaaa ct 152
<210> 41
   <211> 152
   <212> DNA
   <213> Artificial Sequence
<220>
   <223> Chemically modified sequences
<400> 41
   gaagataagt ttatgtattt tgagtttttt tagttgttat ttgtgtgtgg tgatattaaa 60
   gtagagtttt tttataaata gaataagatg ttaaaaaagg tttgtatttt atttttattg 120
   ggagaaatat ttaaaataag gatagattaa aa 152
<210> 42
   <211> 150
   <212> DNA
   <213> Artificial Sequence
<220>
   <223> Chemically modified sequences
<400> 42
   caaattcata tactttaaat tccctcaacc attacctata tataataata tcaaaataaa 60
   attcttccac aaacaaaaca aaatactaaa aaaaatttat actttacttt cattaaaaaa 120
   aatatccaaa ataaaaacaa attaaaaact 150
<210> 43
   <211> 157
   <212> DNA
   <213> Artificial Sequence
<220>
   <223> Chemically modified sequences
<400> 43
   ggagaaaagt attggttggt tttgttttta tttgttttgt taagattatt ttttgttaaa 60
   gtaagtatta gatatttttt gttattattt gtatgtttta tattgtaaat gttattgatt 120
   ttttgattat atagattttt attttttatt ttttttg 157
<210> 44
   <211> 160
   <212> DNA
   <213> Artificial Sequence
<220>
   <223> Chemically modified sequences
<400> 44
   actaacctta aaatttaaaa aaaaatatca attaacttta tctttattta ctttatcaaa 60
   atcatttttt attaaaataa atactaaata ttccttatca ttatctacac actctatact 120
   acaaatacta tcaatttctt aatcacataa acttccattt 160
<210> 45
   <211> 143
   <212> DNA
   <213> Artificial Sequence
<220>
   <223> Chemically modified sequences
<400> 45
   gtgtgtaggg tgaagtgtga gtttattttg ttagttagtg ataggtaggg gtggggggtt 60
   tgttgaggat tttttagttt gatgatgttg atgaatttag gttttagatt gttgttatta 120
   ttggtattgt ataggtatag tgg 143
<210> 46
   <211> 165
   <212> DNA
   <213> Artificial Sequence
<220>
   <223> Chemically modified sequences
<400> 46
   ccaaaactaa ataaaactcc aaatatatat aaaacaaaat ataaatctac cttactaacc 60
   aataacaaac aaaaacaaaa aacccatcaa aaactctcca acttaacaat actaataaat 120
   ccaaactcca aattatcatc atcactaaca ttacacaaat acaac 165
<210> 47
   <211> 133
   <212> DNA
   <213> Artificial Sequence
<220>
   <223> Chemically modified sequences
<400> 47
   ggagaatgtt agtttgagtt aggttttttt gttttgaata tgagtttttt atggtgggag 60
   gtagattgat tttttttgga ttttaggtgg ttgtaggaga tagaagtagg gaggagagat 120
   gatattatat gag 133
<210> 48
   <211> 156
   <212> DNA
   <213> Artificial Sequence
<220>
   <223> Chemically modified sequences
<400> 48

### References

1. Garraway LA & Lander ES (2013) Lessons from the cancer genome. Cell 153(1):17-37.
2. Stratton MR, Campbell PJ, & Futreal PA (2009) The cancer genome. Nature 458(7239):719-724.
3. Vogelstein B, et al. (2013) Cancer genome landscapes. Science 339(6127):1546-1558.
4. Sidransky D, et al. (1992) Identification of ras oncogene mutations in the stool of patients with curable colorectal tumors. Science 256(5053):102-105.
5. Sidransky D, et al. (1991) Identification of p53 gene mutations in bladder cancers and urine samples. Science 252(5006):706-709.
6. Hruban RH, van der Riet P, Erozan YS, & Sidransky D (1994) Brief report: molecular biology and the early detection of carcinoma of the bladder--the case of Hubert H. Humphrey. N Engl J Med 330(18):1276-1278.
7. Tie J, et al. (2016) Circulating tumor DNA analysis detects minimal residual disease and predicts recurrence in patients with stage II colon cancer. Sci Transl Med 8(346):346ra392.
8. Dawson SJ, et al. (2013) Analysis of circulating tumor DNA to monitor metastatic breast cancer. N Engl J Med 368(13):1199-1209.
9. Bettegowda C, et al. (Detection of circulating tumor DNA in early- and late-stage human malignancies. Sci Transl Med 6(224):224ra224.
10. Kinde I, et al. (2013) Evaluation of DNA from the Papanicolaou test to detect ovarian and endometrial cancers. Sci Transl Med 5(167):167ra164.
11. Wang Y, et al. (2015) Detection of somatic mutations and HPV in the saliva and plasma of patients with head and neck squamous cell carcinomas. Sci Transl Med 7(293):293ra104.
12. Wang Y, et al. (2015) Detection of tumor-derived DNA in cerebrospinal fluid of patients with primary tumors of the brain and spinal cord. Proc Natl Acad Sci U S A 112(31):9704-9709.
13. Wang Y, et al. (2016) Diagnostic potential of tumor DNA from ovarian cyst fluid. Elife 5.
14. Springer S, et al. (2015) A combination of molecular markers and clinical features improve the classification of pancreatic cysts. Gastroenterology 149(6):1501-1510.
15. Forshew T, et al. (2012) Noninvasive identification and monitoring of cancer mutations by targeted deep sequencing of plasma DNA. Sci Transl Med 4(136):136ra168.
16. De Mattos-Arruda L & Caldas C (2016) Cell-free circulating tumour DNA as a liquid biopsy in breast cancer. Mol Oncol 10(3):464-474.
17. Vogelstein B & Kinzler KW (1999) Digital PCR. Proc Natl Acad Sci U S A 96(16):9236-9241.
18. Dressman D, Yan H, Traverso G, Kinzler KW, & Vogelstein B (2003) Transforming single DNA molecules into fluorescent magnetic particles for detection and enumeration of genetic variations. Proc Natl Acad Sci U S A 100(15):8817-8822.
19. Margulies M, et al. (2005) Genome sequencing in microfabricated high-density picolitre reactors. Nature 437(7057):376-380.
20. Mitra RD & Church GM (1999) In situ localized amplification and contact replication of many individual DNA molecules. Nucleic Acids Res 27(24):e34.
21. Shendure J & Ji H (2008) Next-generation DNA sequencing. Nat Biotechnol 26(10):1135-1145.
22. Do H & Dobrovic A (2012) Dramatic reduction of sequence artefacts from DNA isolated from formalin-fixed cancer biopsies by treatment with uracil- DNA glycosylase. Oncotarget 3(5):546-558.
23. Do H, Wong SQ, Li J, & Dobrovic A (2013) Reducing sequence artifacts in amplicon-based massively parallel sequencing of formalin-fixed paraffin-embedded DNA by enzymatic depletion of uracil-containing templates. Clin Chem 59(9):1376-1383.
24. Bratman SV, Newman AM, Alizadeh AA, & Diehn M (2015) Potential clinical utility of ultrasensitive circulating tumor DNA detection with CAPP-Seq. Expert Rev Mol Diagn 15(6):715-719.
25. Bokulich NA, et al. (2013) Quality-filtering vastly improves diversity estimates from Illumina amplicon sequencing. Nat Methods 10(1):57-59.
26. Sykes PJ, et al. (1992) Quantitation of targets for PCR by use of limiting dilution. Biotechniques 13(3):444-449.
27. Kinde I, Wu J, Papadopoulos N, Kinzler KW, & Vogelstein B (2011) Detection and quantification of rare mutations with massively parallel sequencing. Proc Natl Acad Sci U SA 108(23):9530-9535.
28. Casbon JA, Osborne RJ, Brenner S, & Lichtenstein CP (2011) A method for counting PCR template molecules with application to next-generation sequencing. Nucleic Acids Res 39(12):e81.
29. Schmitt MW, et al. (2012) Detection of ultra-rare mutations by next-generation sequencing. Proc Natl Acad Sci U S A 109(36):14508-14513.
30. Hoang ML, et al. (2016) Genome-wide quantification of rare somatic mutations in normal human tissues using massively parallel sequencing. Proc Natl Acad Sci U S A 113(35):9846-9851.
31. He Y, Vogelstein B, Velculescu VE, Papadopoulos N, & Kinzler KW (2008) The antisense transcriptomes of human cells. Science 322(5909):1855-1857.
32. Frommer M, et al. (1992) A genomic sequencing protocol that yields a positive display of 5-methylcytosine residues in individual DNA strands. Proc Natl Acad Sci U S A 89(5):1827-1831.
33. Levy D & Wigler M (2014) Facilitated sequence counting and assembly by template mutagenesis. Proc Natl Acad Sci U S A 111(43):E4632-4637.
34. Hayatsu H, Wataya Y, Kai K, & Iida S (1970) Reaction of sodium bisulfite with uracil, cytosine, and their derivatives. Biochemistry 9(14):2858-2865.
35. Clark SJ, Statham A, Stirzaker C, Molloy PL, & Frommer M (2006) DNA methylation: bisulphite modification and analysis. Nat Protoc 1(5):2353-2364.
36. Li M, et al. (2009) Sensitive digital quantification of DNA methylation in clinical samples. Nat Biotechnol 27(9):858-863.
37. Lewis F, Maughan NJ, Smith V, Hillan K, & Quirke P (2001) Unlocking the archive--gene expression in paraffin-embedded tissue. J Pathol 195(1):66-71.
38. Koch I, et al. (2006) Real-time quantitative RT-PCR shows variable, assay-dependent sensitivity to formalin fixation: implications for direct comparison of transcript levels in paraffin-embedded tissues. Diagn Mol Pathol 15(3):149-156.
39. Kinde I, et al. (2013) TERT promoter mutations occur early in urothelial neoplasia and are biomarkers of early disease and disease recurrence in urine. Cancer Res 73(24):7162-7167.
40. Krimmel JD, et al. (2016) Ultra-deep sequencing detects ovarian cancer cells in peritoneal fluid and reveals somatic TP53 mutations in noncancerous tissues. Proc Natl Acad Sci U S A 113(21):6005-6010.
41. Chung W, et al. (2011) Detection of bladder cancer using novel DNA methylation biomarkers in urine sediments. Cancer Epidemiol Biomarkers Prev 20(7):1483-1491.
42. Taby R & Issa JP (2010) Cancer epigenetics. CA Cancer J Clin 60(6):376-392.
43. Issa JP (2012) DNA methylation as a clinical marker in oncology. J Clin Oncol 30(20):2566-2568.
44. Harris FR, et al. (2016) Quantification of Somatic Chromosomal Rearrangements in Circulating Cell-Free DNA from Ovarian Cancers. Sci Rep 6:29831.
45. Bozic I, et al. (2013) Evolutionary dynamics of cancer in response to targeted combination therapy. Elife 2:e00747.
46. Fearon ER & Vogelstein B (1990) A genetic model for colorectal tumorigenesis. Cell 61(5):759-767.
47. Prior IA, Lewis PD, & Mattos C (2012) A comprehensive survey of Ras mutations in cancer. Cancer Res 72(10):2457-2467.
48. Shiraishi M & Hayatsu H (2004) High-speed conversion of cytosine to uracil in bisulfite genomic sequencing analysis of DNA methylation. DNA Res 11(6):409-415.
49. Kandoth C, et al. (2013) Mutational landscape and significance across 12 major cancer types. Nature 502(7471):333-339.
50. Leary RJ, et al. (2012) Detection of chromosomal alterations in the circulation of cancer patients with whole-genome sequencing. Sci Transl Med 4(162):162ra154.
51. Wood LD, et al. (2007) The genomic landscapes of human breast and colorectal cancers. Science 318(5853):1108-1113.
52. Macintyre G, Ylstra B, & Brenton JD (2016) Sequencing Structural Variants in Cancer for Precision Therapeutics. Trends Genet 32(9):530-542.

## Claims

1. A method for detection of a mutation in a population of DNA molecules, comprising:
**treating** a population of DNA molecules with bisulfite to convert unmethylated Cytosine bases in the DNA molecules to Uracil bases, thereby forming a population of converted DNA molecules;
**attaching** molecular barcodes to both strands of the population of converted DNA molecules using an excess of target-specific amplification primers attached to molecular barcodes, thereby forming a population of amplified, barcoded, converted DNA molecules,
**wherein** said amplification primers comprise at least four primers, each of which is complementary to one of four ends of said converted DNA molecules;
**amplifying** the amplified, barcoded, converted DNA molecules in an amplification reaction to form families of amplified, barcoded, converted DNA molecules, wherein amplified, barcoded, converted DNA molecules that share the same molecular barcode form a family of DNA molecules;
**subjecting** a plurality of members of the families to sequencing reactions to obtain nucleotide sequences for both strands of said plurality of members of the families; and
**comparing** nucleotide sequences of a plurality of members of a family and identifying families in which the members contain a selected mutation, and comparing nucleotide sequences of two complementary strands of an amplified, barcoded, converted DNA molecule and identifying the selected mutation in two complementary strands.

2. The method of claim 1, wherein the mutation is in a cancer driver gene selected from the group consisting of: NRAS, PIK3RI, PTEN, RNF43, or TP53.

3. The method of claim 1, wherein the step of attaching employs Phusion U Hot Start polymerase.

4. The method of claim 2, wherein the step of attaching employs a primer selected from the group consisting of SEQ ID NOs: 1-31 and 32, optionally wherein at least four primers selected from the group consisting of SEQ ID NO: 1-31 and 32 are employed.

5. The method of claim 2 wherein the step of amplifying creates an amplicon selected from the group consisting of SEQ ID NOs: 33-47 and 48.

6. The method of claim 1, wherein the target-specific amplification primers comprise modified nucleic acid bases or modified internucleotide linkages.

7. The method of claim 1, wherein:
(i) the step of amplifying adds a sample barcode to amplification products in the amplification reaction, wherein the sample barcode identifies the amplification reaction; or
(ii) prior to the step of amplifying, population of amplified, barcoded, converted DNA molecules is distributed into a plurality of subpopulations.

8. The method of claim 1, wherein the selected mutation is a transversion, an insertion or a deletion.

9. The method of any one of claims 1-8, wherein the mutation is a rare mutation or a polymorphism.

10. The method of any one of claims 1-9, wherein the step of comparing nucleotide sequences of a plurality of members of a family comprises identifying families in which >50% of the members contain a selected mutation, e.g., more than 60%, 70%, 80%, or 90% of the members contain a selected mutation.

11. The method of claim 1, wherein the population of DNA molecules is from a dilute patient sample and the selected mutation has been previously identified in a more concentrated patient sample.

12. The method of any one of claims 1-11, wherein the method detects a mutation in an early stage cancer.

13. A method for detecting methylation at a CpG dinucleotide in plus and minus strands simultaneously, comprising:
**treating** a population of DNA molecules with bisulfite to convert unmethylated Cytosine bases in the DNA molecules to Uracil bases, forming a population of converted DNA molecules;
**attaching** molecular barcodes to both strands of the population of converted DNA molecules using an excess of target-specific amplification primers attached to molecular barcodes, forming a population of amplified, barcoded, converted DNA molecules,
**wherein** said amplification primers comprise at least four primers, each of which is complementary to one of four ends of said converted DNA molecules;
**amplifying** the amplified, barcoded, converted DNA molecules in an amplification reaction to form families of amplified, barcoded, converted DNA molecules, wherein amplified, barcoded, converted DNA molecules that share the same molecular barcode form a family of DNA molecules;
**subjecting** a plurality of members of the families to sequencing reactions to obtain nucleotide sequences for both strands of said plurality of members of the families; and
**comparing** nucleotide sequences of a plurality of members of a family and identifying families in which the members contain a methylated C at a CpG dinucleotide, and
comparing nucleotide sequences of two complementary strands of an amplified, barcoded, converted DNA molecule and identifying the methylated C opposite nucleotide G of the CpG dinucleotide.

14. The method of claim 13 wherein:
(i) the step of amplifying adds a sample barcode to amplification products in the amplification reaction, wherein the sample barcode identifies the amplification reaction; or
(ii) the step of comparing nucleotide sequences of a plurality of members of a family comprises identifying families in which >50% of the members contain a selected mutation, e.g., more than 60%, 70%, 80%, or 90% of the members contain a selected mutation.

## Patentansprüche

1. Verfahren zum Nachweis einer Mutation in einer Population von DNA-Molekülen, umfassend:
Behandeln einer Population von DNA-Molekülen mit Bisulfit zum Umwandeln von unmethylierten Cytosinbasen in den DNA-Molekülen in Uracilbasen, wodurch eine Population von umgewandelten DNA-Molekülen gebildet wird;
Anheften von molekularen Strichcodes an beide Stränge der Population von umgewandelten DNA-Molekülen unter Verwendung eines Überschusses an molekulare Strichcodes angehefteter zielspezifischer Amplifikationsprimer, wodurch eine Population von amplifizierten strichcodierten umgewandelten DNA-Molekülen gebildet wird,
wobei die Amplifikationsprimer mindestens vier Primer umfassen, wovon jeder zu einem der vier Enden der umgewandelten DNA-Moleküle komplementär ist;
Amplifizieren der amplifizierten strichcodierten umgewandelten DNA-Moleküle in einer Amplifikationsreaktion zum Bilden von Familien von amplifizierten strichcodierten umgewandelten DNA-Molekülen, wobei amplifizierte strichcodierte umgewandelte DNA-Moleküle, die den gleichen molekularen Strichcode teilen, eine Familie von DNA-Molekülen bilden;
Durchführen von Sequenzierungsreaktionen mit mehreren Mitgliedern der Familien, sodass Nukleotidsequenzen für beide Stränge der mehreren Mitglieder der Familien erhalten werden; und
Vergleichen von Nukleotidsequenzen mehrerer Mitglieder einer Familie und Identifizieren von Familien, bei welchen die Mitglieder eine ausgewählte Mutation enthalten, und Vergleichen von Nukleotidsequenzen von zwei komplementären Strängen eines amplifizierten strichcodierten umgewandelten DNA-Moleküls und Identifizieren der ausgewählten Mutation in zwei komplementären Strängen.

2. Verfahren nach Anspruch 1, wobei sich die Mutation in einem Krebs-antreibenden Gen befindet, das aus der Gruppe bestehend aus den Folgenden ausgewählt ist: NRAS, PIK3RI, PTEN, RNF43 oder TP53.

3. Verfahren nach Anspruch 1, wobei der Schritt des Anheftens Phusion-U-Hot-Start-Polymerase einsetzt.

4. Verfahren nach Anspruch 2, wobei der Schritt des Anheftens einen Primer einsetzt, der aus der Gruppe bestehend aus SEQ ID NOs: 1-31 und 32 ausgewählt wurde, gegebenenfalls wobei mindestens vier Primer eingesetzt werden, die aus der Gruppe bestehend aus SEQ ID NO: 1-31 und 32 ausgewählt wurden.

5. Verfahren nach Anspruch 2, wobei der Schritt des Amplifizierens ein Amplicon erzeugt, das aus der Gruppe bestehend aus SEQ ID Nr.: 33-47 und 48 ausgewählt wurde.

6. Verfahren nach Anspruch 1, wobei die zielspezifischen Amplifikationsprimer modifizierte Nukleinsäurebasen oder modifizierte Verknüpfungen zwischen den Nukleotiden umfassen.

7. Verfahren nach Anspruch 1, wobei:
(i) der Schritt des Amplifizierens einen Probenstrichcode an Amplifikationsprodukte in der Amplifikationsreaktion hinzufügt, wobei der Probenstrichcode die Amplifikationsreaktion identifiziert; oder
(ii) die Population von amplifizierten strichcodierten umgewandelten DNA-Molekülen vor dem Schritt des Amplifizierens auf mehrere Unterpopulationen verteilt wird.

8. Verfahren nach Anspruch 1, wobei es sich bei der ausgewählten Mutation um eine Transversion, eine Insertion oder eine Deletion handelt.

9. Verfahren nach einem der Ansprüche 1-8, wobei es sich bei der Mutation um eine seltene Mutation oder einen Polymorphismus handelt.

10. Verfahren nach einem der Ansprüche 1-9, wobei der Schritt des Vergleichens der Nukleotidsequenzen mehrerer Mitglieder einer Familie Identifizieren von Familien umfasst, bei denen >50 % der Mitglieder eine ausgewählte Mutation enthalten, z.B. mehr als 60 %, 70 %, 80 % oder 90 % der Mitglieder eine ausgewählte Mutation enthalten.

11. Verfahren nach Anspruch 1, wobei die Population von DNA-Molekülen aus einer verdünnten Patientenprobe stammt und die ausgewählte Mutation zuvor in einer konzentrierteren Patientenprobe identifiziert wurde.

12. Verfahren nach einem der Ansprüche 1-11, wobei das Verfahren eine Mutation in einem Krebs im Frühstadium nachweist.

13. Verfahren zum gleichzeitigen Nachweisen von Methylierung an einem CpG-Dinukleotid in Plus- und Minus-Strängen, umfassend:
Behandeln einer Population von DNA-Molekülen mit Bisulfit zum Umwandeln von unmethylierten Cytosinbasen in den DNA-Molekülen in Uracilbasen, sodass eine Population von umgewandelten DNA-Molekülen gebildet wird;
Anheften von molekularen Strichcodes an beide Stränge der Population von umgewandelten DNA-Molekülen unter Verwendung eines Überschusses an molekulare Strichcodes angehefteter zielspezifischer Amplifikationsprimer, sodass eine Population von amplifizierten strichcodierten umgewandelten DNA-Molekülen gebildet wird,
wobei die Amplifikationsprimer mindestens vier Primer umfassen, wovon jeder zu einem der vier Enden der umgewandelten DNA-Moleküle komplementär ist;
Amplifizieren der amplifizierten strichcodierten umgewandelten DNA-Moleküle in einer Amplifikationsreaktion zum Bilden von Familien von amplifizierten strichcodierten umgewandelten DNA-Molekülen, wobei amplifizierte strichcodierte umgewandelte DNA-Moleküle, die den gleichen molekularen Strichcode teilen, eine Familie von DNA-Molekülen bilden;
Durchführen von Sequenzierungsreaktionen mit mehreren Mitgliedern der Familien, sodass Nukleotidsequenzen für beide Stränge der mehreren Mitglieder der Familien erhalten werden; und
Vergleichen von Nukleotidsequenzen mehrerer Mitglieder einer Familie und Identifizieren von Familien, bei welchen die Mitglieder ein methyliertes C bei einem CpG-Dinukleotid enthalten, und
Vergleichen von Nukleotidsequenzen von zwei komplementären Strängen eines amplifizierten strichcodierten umgewandelten DNA-Moleküls und Identifizieren des methylierten C gegenüber dem Nukleotid G des CpG-Dinukleotids.

14. Verfahren nach Anspruch 13, wobei:
(i) der Schritt des Amplifizierens einen Probenstrichcode an Amplifikationsprodukte in der Amplifikationsreaktion hinzufügt, wobei der Probenstrichcode die Amplifikationsreaktion identifiziert; oder
(ii) der Schritt des Vergleichens der Nukleotidsequenzen mehrerer Mitglieder einer Familie Identifizieren von Familien umfasst, bei denen >50 % der Mitglieder eine ausgewählte Mutation enthalten, z.B. mehr als 60 %, 70 %, 80 % oder 90 % der Mitglieder eine ausgewählte Mutation enthalten.

## Revendications

1. Procédé de détection d'une mutation dans une population de molécules d'ADN, comprenant :
le traitement d'une population de molécules d'ADN avec du bisulfite pour convertir les bases cytosines non méthylées des molécules d'ADN en bases uraciles, formant ainsi une population de molécules d'ADN converties ;
la liaison de codes-barres moléculaires aux deux brins de la population de molécules d'ADN converties au moyen d'un excès d'amorces d'amplification spécifiques d'une cible liées aux codes-barres moléculaires, formant ainsi une population de molécules d'ADN amplifiées, marquées par codes-barres et converties,
dans lequel lesdites amorces d'amplification comprennent au moins quatre amorces, chacune étant complémentaire de l'une des quatre extrémités desdites molécules d'ADN converties ;
l'amplification des molécules d'ADN amplifiées, marquées par codes-barres et converties dans une réaction d'amplification pour former des familles de molécules d'ADN amplifiées, marquées par codes-barres et converties, les molécules d'ADN amplifiées, marquées par codes-barres et converties qui partagent le même code-barres moléculaire formant une famille de molécules d'ADN ;
la soumission d'une pluralité de membres des familles à des réactions de séquençage afin d'obtenir les séquences nucléotidiques des deux brins de ladite pluralité de membres des familles ; et
la comparaison des séquences nucléotidiques d'une pluralité de membres d'une famille et l'identification des familles dans lesquelles les membres contiennent une mutation sélectionnée, et la comparaison des séquences nucléotidiques de deux brins complémentaires d'une molécule d'ADN amplifiée, marquée par code-barres et convertie et l'identification de la mutation sélectionnée dans deux brins complémentaires.

2. Procédé selon la revendication 1, dans lequel la mutation est située dans un gène conducteur du cancer choisi parmi : NRAS, PIK3RI, PTEN, RNF43 ou TP53.

3. Procédé selon la revendication 1, dans lequel l'étape de liaison utilise la polymérase Phusion U Hot Start.

4. Procédé selon la revendication 2, dans lequel l'étape de liaison utilise une amorce choisie dans le groupe constitué par SEQ ID NO : 1 à 31 et 32, facultativement, au moins quatre amorces choisies dans le groupe constitué par SEQ ID NO : 1 à 31 et 32 étant utilisées.

5. Procédé selon la revendication 2, dans lequel l'étape d'amplification crée un amplicon choisi dans le groupe constitué par SEQ ID NO : 33 à 47 et 48.

6. Procédé selon la revendication 1, dans lequel les amorces d'amplification spécifiques d'une cible comprennent des nucléobases modifiées ou des liaisons internucléotidiques modifiées.

7. Procédé selon la revendication 1, dans lequel :
(i) l'étape d'amplification ajoute un code-barres d'échantillon aux produits d'amplification lors de la réaction d'amplification, le code-barres d'échantillon identifiant la réaction d'amplification ; ou
(ii) avant l'étape d'amplification, la population de molécules d'ADN amplifiées, marquées par codes-barres et converties est répartie en une pluralité de sous-populations.

8. Procédé selon la revendication 1, dans lequel la mutation sélectionnée est une transversion, une insertion ou une délétion.

9. Procédé selon l'une quelconque des revendications 1 à 8, dans lequel la mutation est une mutation rare ou un polymorphisme.

10. Procédé selon l'une quelconque des revendications 1 à 9, dans lequel l'étape de comparaison des séquences nucléotidiques d'une pluralité de membres d'une famille comprend l'identification de familles dans lesquelles plus de 50 % des membres contiennent une mutation sélectionnée, par exemple, plus de 60 %, 70 %, 80 % ou 90 % des membres contiennent une mutation sélectionnée.

11. Procédé selon la revendication 1, dans lequel la population de molécules d'ADN provient d'un échantillon de patient dilué et la mutation sélectionnée a été précédemment identifiée dans un échantillon de patient plus concentré.

12. Procédé selon l'une quelconque des revendications 1 à 11, le procédé détectant une mutation dans un cancer à un stade précoce.

13. Procédé de détection simultanée de la méthylation d'un dinucléotide CpG sur les brins positif et négatif, comprenant :
le traitement d'une population de molécules d'ADN avec du bisulfite pour convertir les bases cytosines non méthylées des molécules d'ADN en bases uraciles, de façon à former une population de molécules d'ADN converties ;
la liaison de codes-barres moléculaires aux deux brins de la population de molécules d'ADN converties au moyen d'un excès d'amorces d'amplification spécifiques d'une cible liées aux codes-barres moléculaires, de façon à former une population de molécules d'ADN amplifiées, marquées par codes-barres et converties,
dans lequel lesdites amorces d'amplification comprennent au moins quatre amorces, chacune étant complémentaire de l'une des quatre extrémités desdites molécules d'ADN converties ;
l'amplification des molécules d'ADN amplifiées, marquées par codes-barres et converties dans une réaction d'amplification pour former des familles de molécules d'ADN amplifiées, marquées par codes-barres et converties, les molécules d'ADN amplifiées, marquées par codes-barres et converties qui partagent le même code-barres moléculaire formant une famille de molécules d'ADN ;
la soumission d'une pluralité de membres des familles à des réactions de séquençage afin d'obtenir les séquences nucléotidiques des deux brins de ladite pluralité de membres des familles ; et
la comparaison des séquences nucléotidiques d'une pluralité de membres d'une famille et l'identification des familles dans lesquelles les membres contiennent un C méthylé au niveau d'un dinucléotide CpG ; et
la comparaison des séquences nucléotidiques de deux brins complémentaires d'une molécule d'ADN amplifiée, marquée par code-barres et convertie, et l'identification du C méthylé apparié au nucléotide G du dinucléotide CpG.

14. Procédé selon la revendication 13, dans lequel :
(i) l'étape d'amplification ajoute un code-barres d'échantillon aux produits d'amplification lors de la réaction d'amplification, le code-barres d'échantillon identifiant la réaction d'amplification ; ou
(ii) l'étape de comparaison des séquences nucléotidiques d'une pluralité de membres d'une famille comprend l'identification de familles dans lesquelles plus de 50 % des membres contiennent une mutation sélectionnée, par exemple, plus de 60 %, 70 %, 80 % ou 90 % des membres contiennent une mutation sélectionnée.
